# EUROPEAN PATENT APPLICATION

(11) **EP 4 684 801 A1**
(43) Date of publication of application: **28.01.2026**
(21) Application number: 24190449.9
(22) Date of filing: 23.07.2024
(51) Int. Cl.: A61K 47/34, A61K 38/22, A61K 38/26, A61P 3/04, A61P 3/10, A61P 25/28

(54) **COMPOSITION COMPRISING SEMAGLUTIDE, CAGRILINTIDE AND A CO-POLYAMINO ACID BEARING CARBOXYLATE CHARGES AND HYDROPHOBIC RADICALS**

(71) Applicant: Adocia, 69003 Lyon (FR)
(72) Inventor: CHAN, You Ping, 69003 Lyon (FR)
(74) Representative: Casalonga

(57) **Abstract**

The invention relates to a composition comprising semaglutide, cagrilintide and a co-polyamino acid (PLG) bearing carboxylate charges and hydrophobic radicals, said composition being in the form of aqueous injectable solution at pH between 5.0 to 6.0.

## Description

The invention relates to a composition comprising semaglutide, cagrilintide and a co-polyamino acid (PLG) bearing carboxylate charges and hydrophobic radicals, said composition being in the form of aqueous injectable solution at pH between 5.0 to 6.0.

This composition may be used as a medicament, in particular for the treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

Formulating in the same composition semaglutide and cagrilintide was not considered possible, due to different physicochemical properties of these active pharmaceutical ingredients (APIs) until WO2123/187067. This application from Novo Nordisk proposes a solution to co-formulate said APIs by using a cyclodextrin comprising hydroxypropyl substitution.

The compositions of WO2023/187067 prior art needs high concentration of cyclodextrins to solubilize semaglutide. Furthermore, compositions of the prior art are not sufficiently stable, in particular regarding the level of impurities.

A goal of the invention is to allow an improved stability of of semaglutide and cagrilintide in a composition having pH between 5.0 to 6.0.

Therefore, there is a need to find a solution for obtaining stable compositions comprising semaglutide and cagrilintide.

Specifically, one of the goals of the present invention is to enhance the chemical stability of cagrilintide while having a good stability of semaglutide.

Among problems to be solved with peptides as APIs can be cited improving their efficacy, their duration of action (t half-life), in particular for those having a long half-life, and decreasing their undesirable side-effect(s).

Another problem to be solved is to be able to obtain a solution which comprises preservatives, in particular such as phenol or m-cresol, with a limited amount of excipients.

Among the problems to be solved can also be cited the improvement of local tolerance and comfort upon injection.

Another problem to be solved is to use less amount of the excipient (co-polyamino acid) allowing the co-formulation.

Therefore, there is a need to find a solution for solving at least in part the problems cited above.

Surprisingly, the applicant has found that a composition, in the form of an aqueous injectable solution, comprising semaglutide and cagrilintide with a co-polyamino acids bearing carboxylate charges and hydrophobic radicals allows to solve at least in part the above cited problems.

The invention relates to a composition, in the form of an aqueous injectable solution, whose pH is comprised from 5.0 to 6.0, in particular from 5.1 to 5.9, comprising:
- semaglutide
- cagrilintide, and
- a co-polyamino acid (PLG) bearing carboxylate charges and hydrophobic radicals Hy, said co-polyamino acid consisting of glutamic or aspartic units, and said hydrophobic radicals Hy being according to formula X below: 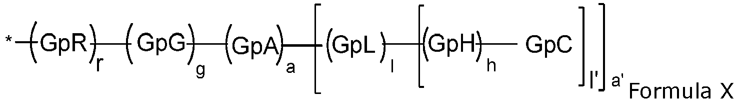 in which
- GpR is chosen from the radicals according to formulas VII, VII' or VII': 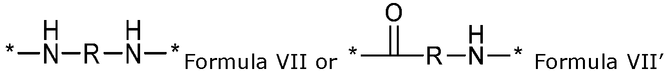 or 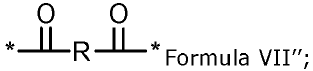 identical or different GpG and GpH are chosen from the radicals according to formulas XI or XI': 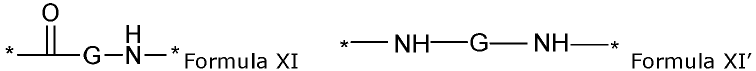
- GpA is chosen from the radicals according to formula VIII:

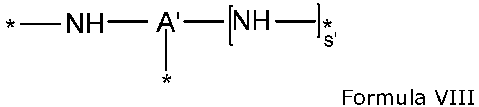

∘ in which A' is chosen from the radicals according to formula VIII', VIII" or VIII‴:

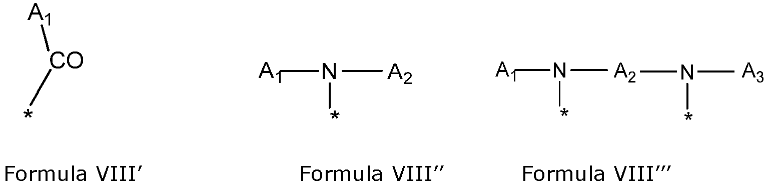

- GpL is chosen from the radicals according to formula XII: 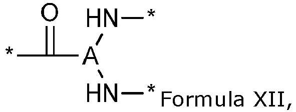
- GpC is a radical according to formula IX: 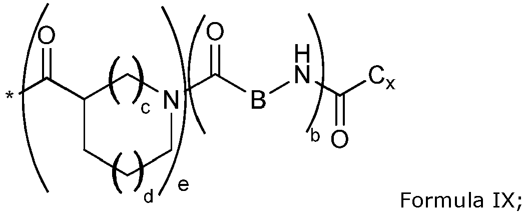 the * indicates the attachment sites of the different groups bonded by the amide functions;
- a is an integer equal to 0 or to 1 and a' = 1 if a = 0 and a' = 1, 2 or 3 if a = 1;
- a' is an integer equal to 1, to 2, or to 3
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- l is an integer equal to 0 or 1 and I' = 1 if I = 0, and I' = 2 if I = 1;
- r is an integer equal to 0, to 1, or to 2, and
- s' is an integer equal to 0 or 1, and
- if e is different from 0, then at least one of g, h or I is different from 0;
- A, A₁, A₂ and A₃, identical or different, are linear or branched alkyl radicals, and/or substituted by a radical derived from a saturated, unsaturated or aromatic ring, comprising 1 to 8 carbon atoms,
- B is a linear or branched alkyl radical and/or comprising an aromatic nucleus, comprising 1 to 9 carbon atoms or an unsubstituted ether or polyether radical comprising from 4 to 14 carbon atoms and from 1 to 5 oxygen atoms;
- Cₓ is a linear or branched monovalent alkyl radical, and/or comprising a cyclic part, in which x indicates the number of carbon atoms and:
   ∘ when the hydrophobic radical -Hy carries 1 -GpC, then 9 ≤ x ≤ 25
   ∘ when the hydrophobic radical -Hy carries 2 -GpC, then 9 ≤ x ≤ 15,
   ∘ when the hydrophobic radical -Hy carries 3 -GpC, then 7 ≤ x ≤ 13,
   ∘ when the hydrophobic radical -Hy carries 4 -GpC, then 7 ≤ x ≤ 11,
   ∘ when the hydrophobic radical -Hy carries at least 5 -GpC, then 6 ≤ x ≤ 11,
- G is a branched alkyl radical of 1 to 8 carbon atoms, with said alkyl radical bearing one or more free carboxylic acid functions,
- R is a radical chosen from the group consisting of a divalent alkyl radical, linear or branched, comprising 1 to 12 carbon atoms, a divalent alkyl radical, linear or branched, comprising 1 to 12 carbon atoms bearing one or more -CONH₂ functions, or an unsubstituted ether or polyether radical comprising from 4 to 14 carbon atoms and 1 to 5 oxygen atoms:
- the hydrophobic radical(s) -Hy according to formula X being bonded to the PLG:
   ∘ via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
   ∘ via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0 < M ≤ 0.5;
- when several hydrophobic radicals are borne by a co-polyamino acid, then they are identical or different,
- the degree of polymerization DP in glutamic or aspartic units for PLG chains is comprised from 5 to 250;
- free carboxylic acid functions being in the form of an alkali metal salt chosen from the group consisting of Na⁺ and K⁺.

The invention also concerns compositions for use as a medicament.

The invention also concerns compositions for use in a method of treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

The invention also concerns an injection device comprising a composition according to the invention.

The invention also concerns a container, such as a vial or a cartridge comprising a composition according to the invention.

The invention also concerns a method for the preparation of a stable, injectable aqueous solutions of semaglutide comprising the step of mixing a co-polyamino acid with the semaglutide.

### DEFINITIONS

By "physically stable composition" is meant compositions that meet the visual inspection criteria described in European, American and international pharmacopoeia, that is, compositions that are clear and that do not contain visible particles and are also colorless.

The "chemical stability" can be evaluated by RP-HPLC or RP-UPLC for estimating peptide purity and SEC-HPLC for estimating high molecular weight products (HMWP).

By "injectable aqueous solution" is meant solutions for which the solvent is water and which meets the pharmacopoeia conditions of Europe and the US.

Compositions in the form of an injectable aqueous solution according to the invention are clear solutions. By "clear solution" is meant compositions that meet the criteria described in American and European pharmacopoeia regarding injectable solutions. In US pharmacopoeia, solutions are defined in part <1151>, referring to injection <1> (referring to <788> according to USP 35 and specified in <788> according to USP 35 and in <787>, <788> and <790> USP 38 (beginning on August 1st, 2014), according to USP 38). In European pharmacopoeia, injectable solutions must meet the criteria provided in sections 2.9.19 and 2.9.20.

By "co-polyamino acid being constituted of glutamic or aspartic units" is meant linear, non-cyclic units of glutamic or aspartic acids bonded to each other by peptidic bonds, said chains presenting a C-terminal part, corresponding to carboxylic acid at one end, and an N-terminal part, corresponding to amine at the other end of the chain. The N-terminal part and the C-terminal part may be substituted, in particular as defined in Formulae XXXa, XXXa', XXXb, XXXb' and A-XXX.

In the following, and without other indication, the limits of a value range are included within this range, particularly in the expressions "between" and "ranging from ... to ...".

### Co-polyamino acid

Said co-polyamino acid bearing carboxylate charges and hydrophobic -Hy radicals is soluble in an aqueous solution of pH between 5.0 and 6.0, at a temperature of 25 °C and at a concentration of 50 mg/ml.

Said co-polyamino acid bearing carboxylate charges and hydrophobic -Hy radicals is soluble in an aqueous solution of pH between 5.0 and 6.0, at a temperature of 25 °C and at a concentration of 20 mg/ml.

The co-polyamino acid is a random co-polyamino acid in the chain of glutamic and/or aspartic units.

In a preferred embodiment the co-polyamino acid is the chain of glutamic units.

By "alkyl radical" is meant a carbon-comprising chain, linear or branched, which does not include a heteroatom.

In the formulas, the *s indicate the attachment sites of the various elements represented.

The radicals Hy, GpR, GpG, GpH, GpA, GpL and GpC are each independently identical or different from one residue to another.

In one embodiment, the composition according to the invention is characterized in that the Hy comprises between 15 and 100 atoms of carbon.

In one embodiment, the composition according to the invention is characterized in that the Hy comprises between 30 and 70 atoms of carbon.

In one embodiment, the composition according to the invention is characterized in that the Hy comprises between 40 and 60 atoms of carbon.

In one embodiment, the composition according to the invention is characterized in that the Hy comprises between 20 and 30 atoms of carbon.

In one embodiment, the composition according to the invention is characterized in that the Hy comprises more than 30 atoms of carbon.

In the formulas, the *s indicate the attachment sites of hydrophobic radicals to the PLG or between the different GpR, GpG, GpH, GpA, GpL and GpC to form amide functions.

Hy radicals are attached to the PLG via amide functions.

In one embodiment, r=0 and the hydrophobic radical of formula X is bonded to the PLG via a covalent bond between a carbonyl of the hydrophobic radical and a nitrogen atom borne by the PLG, thus forming an amide function resulting from the reaction of an amine function borne by the PLG precursor and and acid function borne by the -Hy' precursor of the hydrophobic radical.

In one embodiment, r=1 or 2 and the hydrophobic radical of formula X is bonded to the PLG:
▪ via a covalent bond between a nitrogen atom of the hydrophobic radical and a carbonyl borne by the PLG, thus forming an amide function resulting from the reaction of an amine function of the -Hy' precursor of the hydrophobic radical and an acid function borne by the PLG, or
▪ via a covalent bond between a carbonyl of the hydrophobic radical and a nitrogen atom borne by the PLG, thus forming an amide function resulting from the reaction of an amine function of the Hy' precursor of the hydrophobic -Hy radical and an acid function borne by the PLG.

In one embodiment, if GpA is a radical of formula VIIIc and r=1, then:
- the GpCs are directly or indirectly bonded to N_{α1} and N_{α2} and the PLG is directly or indirectly bonded via GpR to N_{β1} , or
- the GpCs are directly or indirectly bonded to N_{α1} and N_{β1} and the PLG is directly or indirectly bonded via GpR to N_{α2} , or
- the GpCs are directly or indirectly bonded to N_{α2} and N_{β1} and the PLG is directly or indirectly bonded via GpR to N_{α1}.

In one embodiment, if GpA is a radical of formula VIIIc and r=0, then:
- the GpCs are directly or indirectly bonded to N_{α1} and N_{α2} and the PLG is directly or indirectly bonded to N_{β1}; or
- the GpCs are directly or indirectly bonded to N_{α1} and N_{β1} and the PLG is directly or indirectly bonded to N_{α2}; or
- the GpCs are directly or indirectly bonded to N_{α2} and N_{β1}, and the PLG is directly or indirectly bonded to N_{α1}.

In one embodiment, if GpA is a radical of formula VIIId and r=1, then
- the GpCs are directly or indirectly bonded to N_{α1}, N_{α2} and N_{β1} and the PLG is directly or indirectly bonded via GpR to N_{β2}, or
- the GpCs are directly or indirectly bonded to N_{α1}, N_{α2} and N_{β2} and the PLG is directly or indirectly bonded via GpR to N_{β1}, or
- the GpCs are directly or indirectly bonded to N_{α1}, N_{β1} and N_{β2} and the PLG is directly or indirectly bonded via GpR to N_{α2}, or
- the GpCs are directly or indirectly bonded to N_{α2}, N_{β1} and N_{β2} and the PLG is directly or indirectly bonded via GpR to N_{α1}.

In one embodiment, if GpA is a radical of formula VIIId and r=0, then
- the GpCs are directly or indirectly bonded to N_{α1}, N_{α2} and N_{β1} and the PLG is directly or indirectly bonded to N_{β2}; or
- the GpCs are directly or indirectly bonded to N_{α1}, N_{α2} and N_{β2} and the PLG is directly or indirectly bonded to N_{β1}; or
- the GpCs are directly or indirectly bonded to N_{α1}, N_{β1} and N_{β2} and the PLG is directly or indirectly bonded to N_{α2}; or
- the GpCs are directly or indirectly bonded to N_{α2}, N_{β1} and N_{β2} and the PLG is directly or indirectly bonded to N_{α1.*}.

In one embodiment, when r=2, then the GpR group bonded to the PLG is chosen from among the GpRs of formula VII.

In one embodiment, when r=2, then the first GpR group bonded to the PLG is chosen from among the GpRs of formula VII and the second GpR is chosen from among the GpRs of formula VII".

In one embodiment, an embodiment, when r=2 then the GpR bonded to the PLG is chosen from among the GpRs of formula VII".

In one embodiment, an embodiment, when r=2, then the first GpR group bonded to the PLG is chosen from among the GpRs of formula VII" and the second GpR is chosen from among the GpRs of formula VII.

In one embodiment, a=0,

In one embodiment, h=1 and g=0,

In one embodiment, h=0 and g=1,

In one embodiment, r=0, g=1 and h=0.

In one embodiment, at least one of g, h or I is different from 0.

In one embodiment, at least one of g and h is equal to 1.

In one embodiment, a=1 and I=1.

In one embodiment, if I=0, at least one of g or h is equal to 0.

In one embodiment, if I=1, at least one of g or h is equal to 0.

In one embodiment, g+h≥2.

In one embodiment, g is greater than or equal to 2 (g≥2).

In one embodiment, h is greater than or equal to 2 (h≥2).

In one embodiment, g+h≥2 and a and I are equal to 0 (a=I=0).

In one embodiment, g+h≥2 and b is equal to 0 (b=0).

In one embodiment, g is greater than or equal to 2 (g≥2) and b is equal to 0.

In one embodiment, g+h≥2, b is equal to 0 (b=0) and e is equal to 1 (e=1).

In one embodiment, g or h is greater than or equal to 2 (g≥2) b is equal to 0 (b=0) and e is equal to 1 (e=1).

In a most preferred embodiment, said at least one hydrophobic radical -Hy is chosen from among the radicals of formula X wherein
- GpR is according to Formula VII, with r = 0 or 1
- g = 0
- GpA is according to Formula VIII with s' = 1, A' is according to Formula VIII' with A1 being a linear alkyl radical comprising from 2 to 5 carbon atoms, in particular GpA corresponds to a Lysine residue trisubstituted, bonded to either GpR or the terminal -NH- of the PLG via its -CO-, thus forming an amide bond and bonded to GpH via its -NH-, thus forming an amide bond,
- I = 0
- GpH is according to Formula XI, with h = 1, 2 or 3, in particular 1,
- GpC with e = 1, c = 0, d = 1, b = 0 and x being comprised between 13 to 15,
- I' = 1 and a' = 2.

In one embodiment, said at least one hydrophobic radical -Hy is chosen from among the radicals of formula X in which r=2 of formula Xc', as defined below: 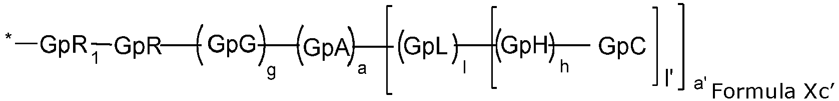 in which GpR₁ is a radical from formula VII. 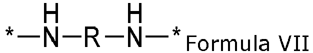 in which GpR, GpG, GpA, GpL, GpH, GpC, R, a, a', g, h, I and I' have the definitions given above.

In one embodiment, said at least one hydrophobic radical -Hy is chosen from among the radicals of formula X in which r=2 of formula Xc', as defined below: 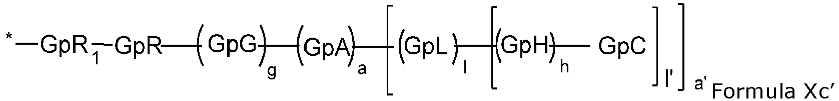 in which GpR₁, is a radical from formula VII". 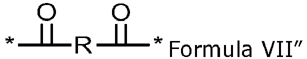 in which GpR, GpG, GpA, GpL, GpH, GpC, R, a, a', g, h, I and I' have the definitions given above.

In one embodiment, g=h=0, a=1, GpA is a radical from Formula VIII with s'=1 and A' from Formula VIII' or VIII", and I=1.

In one embodiment, said at least one hydrophobic radical -Hy is chosen from among the radicals of formula X in which
- I=0,
- from formula Xb' as defined below. 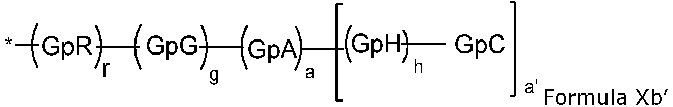 in which
- GpR is chosen from among the radicals of formulas VII, VII' or VII": 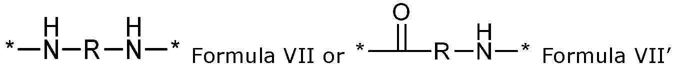 or 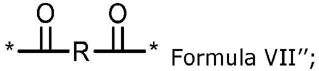
- GpG is chosen from among the radicals of formula XI or XI': 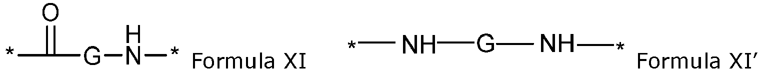
- GpA is chosen from among the radicals of formula VIII in which s'=1 represented by formula VIIIa or formula VIII in which and s'=0 represented by formula VIIIb 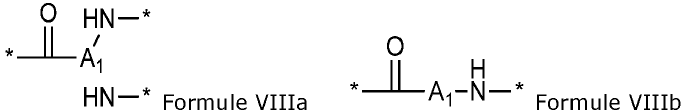
- GpC is a radical from formula IX: 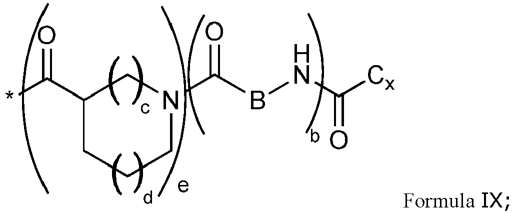
- The *s indicate the attachment sites of the different groups bonded by the amide functions;
- a is an integer equal to 0 or to 1 and a' = 1 if a = 0'= if and a'=1 or a'=2 if a = 1;
- a' is an integer equal to 1 or 2 and
   ∘ If a' is equal to 1 then a is equal to 0 or to 1 and GpA is a radical from formula VIIIb and,
   ∘ if a' is equal to 2 then a is equal to 1 and GpA is a radical from formula VIIIa;
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6, and at least one of g or h is different from 0;
- r is an integer equal to 0, to 1, or to 2, and
- s' is a whole number equal to 0 or 1;
- A₁ is a linear or branched alkyl radical, and possibly substituted by a radical from a saturated, unsaturated or aromatic ring, comprising 1 to 6 carbon atoms.
- B is a linear or branched alkyl radical possibly comprising an aromatic core, comprising 1 to 9 carbon atoms or a non-substituted ether or polyether radical comprising 4 to 14 carbon atoms and 1 to 5 oxygen atoms;
- Cₓ is a linear or branched monovalent alkyl radical, possibly comprising a ring part, in which x indicates the number of carbon atoms and:
   ▪ When the hydrophobic radical -Hy carries 1 -GpC, then 9 ≤ x ≤ 25,
   ▪ When the hydrophobic radical -Hy carries 2 -GpC, then 9 ≤ x ≤ 15,
   ▪ When the hydrophobic radical -Hy carries 3 -GpC, then 7 ≤ x ≤ 13,
   ▪ When the hydrophobic radical -Hy carries 4 -GpC, then 7 ≤ x ≤ 11,
   ▪ When the hydrophobic radical -Hy carries at least 5 -GpC, then 6 ≤ x ≤ 11,
- G is a branched alkyl radical of 1 to 8 carbon atoms, with said alkyl radical bearing one or more free carboxylic acid functions,
- R is a radical chosen from the group constituted by a divalent alkyl radical, linear or branched, comprising 1 to 12 carbon atoms, a divalent alkyl radical, linear or branched, comprising 1 to 12 carbon atoms bearing one or more - CONH₂ functions, or a non-substituted ether or polyether radical comprising from 4 to 14 carbon atoms and 1 to 5 oxygen atoms:
   - The hydrophobic Hy radical(s) of formula X being bonded to the PLG:
      ∘ via a covalent bond between a carbonyl of the hydrophobic radical and a nitrogen atom borne by the PLG, thus forming an amide function resulting from the reaction of an amine function borne by the PLG and and acid function borne by the precursor of the hydrophobic, or
      ∘ via a covalent bond between a nitrogen atom of the hydrophobic radical and a carbonyl borne by the PLG. Thus forming an amide function resulting from the reaction of an amine function of the -Hy' precursor of the hydrophobic radical and an acid function borne by the PLG,
   - the ratio M between the number of hydrophobic radicals and the number of glutamic or aspartic units being between 0 < M ≤ 0,5;
   - when several hydrophobic radicals are borne by a co-polyamino acid, then they are identical or different,
   - free carboxylic acid functions being in the form of an alkali metal salt chosen from the group constituted by Na⁺ and K⁺.

In one embodiment said at least one hydrophobic radical -Hy is chosen from among the radicals of formula X as defined below in which I= 0,
- GpA is chosen from among the radicals in formula VIII in which s'=1 and A' is chosen from among the radicals of formula VIII" or VIII'",
- from formula Xb' as defined below: 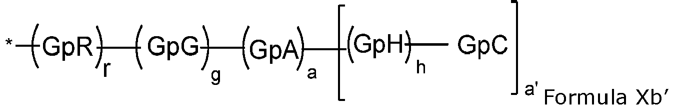 in which
- GpR is chosen from among the radicals of formulas VII, VII' or VII": 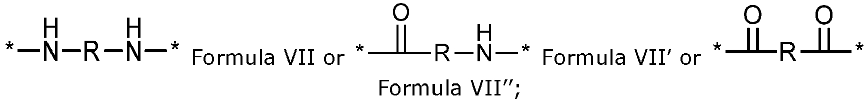
- GpG is chosen from among the radicals of formula XI or XI': 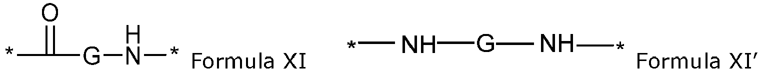
- GpA is chosen from among the radicals of formulas VIIIc or VIIId: 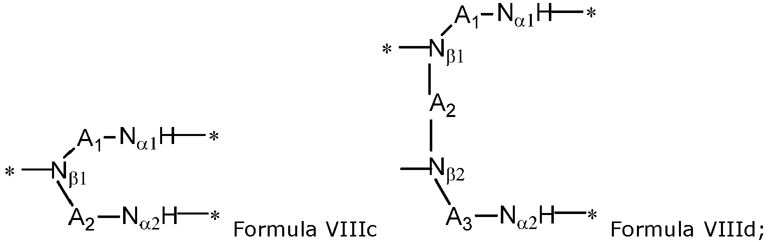
- GpC is a radical from formula IX: 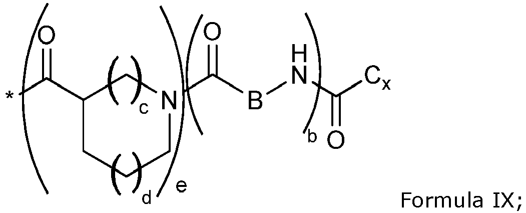
- The *s indicate the attachment sites of the different groups bonded by the amide functions;
- a is a whole equal to 0 or to 1 and a' = 1 if a = 0, and a' = 2 or 3 if a = 1;
- a' is an integer equal to 2 or 3 and
   ∘ if a' is equal to 1 then a is equal to 0 and,
   ∘ if a' is equal to 2 or 3 then a is equal to 1 and GpA is a radical from formula VIIIc or VIIId;
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6, and at least one of g or h is different from 0;
- r is whole number equal to 0, to 1, or to 2, and
- s' is an integer equal to 1;
- A₁, A₂, A₃ identical or different, are linear or branched alkyl radicals, and possibly substituted by a radical from a saturated, unsaturated or aromatic ring, comprising 1 to 6 carbon atoms.
- B is a linear or branched alkyl radical possibly comprising an aromatic core, comprising 1 to 9 carbon atoms or a non-substituted ether or polyether radical comprising 4 to 14 carbon atoms and 1 to 5 oxygen atoms;
- Cₓ is a linear or branched monovalent alkyl radical, possibly comprising a ring part, in which x indicates the number of carbon atoms and:
   ▪ When the hydrophobic radical -Hy carries 1 -GpC, then 9 ≤ x ≤ 25,
   ▪ When the hydrophobic radical -Hy carries 2 -GpC, then 9 ≤ x ≤ 15,
   ▪ When the hydrophobic radical -Hy carries 3 -GpC, then 7 ≤ x ≤ 13,
   ▪ When the hydrophobic radical -Hy carries 4 -GpC, then 7 ≤ x ≤ 11,
   ▪ When the hydrophobic radical -Hy carries 5 -GpC, then 6 ≤ x ≤ 11,
- The hydrophobic Hy radical(s) of formula X being bonded to the PLG:
   ∘ via a covalent bond between a carbonyl of the hydrophobic radical and a nitrogen atom borne by the PLG, thus forming an amide function resulting from the reaction of an amine function borne by the PLG and and acid function borne by the -Hy' precursor of the hydrophobic radical,
      or
   ∘ via a covalent bond between a nitrogen atom of the hydrophobic radical and a carbonyl borne by the PLG. Thus forming an amide function resulting from the reaction of an amine function of the -Hy' precursor of the hydrophobic radical and an acid function borne by the PLG,
- G is a branched alkyl radical of 1 to 8 carbon atoms, with said alkyl radical bearing one or more free carboxylic acid functions,
- R is a radical chosen from the group constituted by a divalent alkyl radical, linear or branched, comprising 1 to 12 carbon atoms, a divalent alkyl radical, linear or branched, comprising 1 to 12 carbon atoms bearing one or more -CONH₂ functions, or a non-substituted ether or polyether radical comprising from 4 to 14 carbon atoms and 1 to 5 oxygen atoms:
   - the ratio M between the number of hydrophobic radicals and the number of glutamic or aspartic units being between 0 < M ≤ 0.5;
   - when several hydrophobic radicals are borne by a co-polyamino acid, then they are identical or different,
   - free carboxylic acid functions being in the form of an alkali metal salt chosen from the group constituted by Na⁺ and K⁺.

In one embodiment, said at least one hydrophobic radical -Hy is chosen from among the radicals of formula X in which a=1 and a'=1 of formula Xa, as defined below: 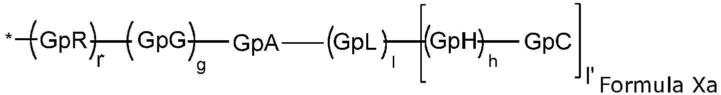 in which GpA is a radical of formula VIII and A' is chosen from among the radicals of formula VIII' with s'=0 and GpA is a radical of Formula VIIIb 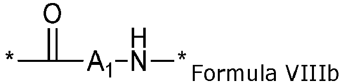 and GpR, GpG, GpL, GpH, GpC, A₁, r, g, h, I and I' have the definitions given above.

In one embodiment, said at least one hydrophobic radical -Hy is chosen from among the radicals of formula X in which a=1 of formula Xb, as defined below: 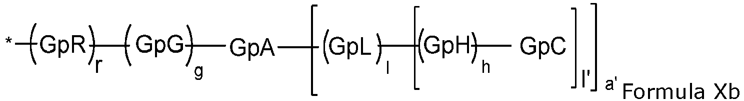 in which GpA is a radical of formula VIII and A' is chosen from among the radicals of formula VIII' with s'=1 and GpA is a radical of Formula VIIIa 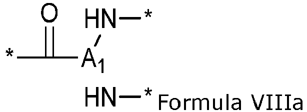 and GpR, GpG, GpL, GpH, GpC, A₁, a', r, g, h, I and I' have the definitions given above.

In one embodiment, said at least one hydrophobic radical -Hy is chosen from among the radicals of formula X in which a=1, as defined below: 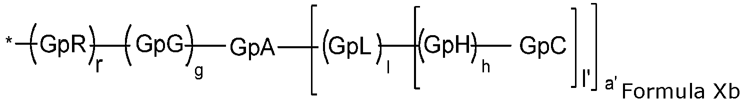 in which GpA is a radical from formula VIII and A is chosen from among the radicals of Formula VIII" with s'=1 and GpA is a radical of Formula VIIIc 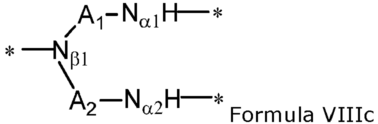 and GpR, GpG, GpL, GpH, GpC, A₁, A₂, r, g, h, a', I and I' have the definitions given above.

In one embodiment, said at least one hydrophobic radical -Hy is chosen from among the radicals of formula X in which a=1, as defined below: 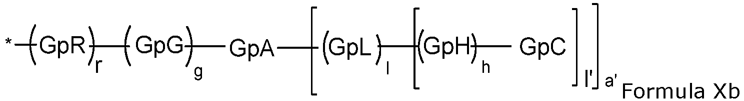 in which GpA is a radical of formula VIII and A is chosen from among the radicals of formula VIII‴ with s'=1, and GpA is a radical of Formula VIIId 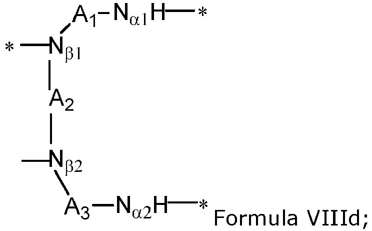 and GpR, GpG, GpL, GpH, GpC, A₁, A₂, A₃, a', r, g, h, I and I' have the definitions given above.

In one embodiment, the composition according to the invention is characterized in that said hydrophobic radicals are chosen from among the hydrophobic radicals of formula X in which GpA is a radical from formula VIIIb, a'=1 and I=0, represented by the following formula Xe: 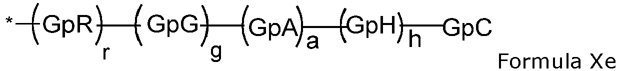 GpR, GpG, GpA, GpH, GpC, r, g, h, and a have the definitions given above.

In one embodiment, r=0, and GpA is chosen from among the radicals of formulas VIIIa and VIIIb.

In one embodiment, r=0, g=0 and GpA is chosen from among the radicals of formula VIIIa and VIIIb.

In one embodiment, r=0, and GpA is chosen from among the radicals of formulas VIIIa and VIIIb and h=0.

In one embodiment, said at least one hydrophobic radical -Hy is chosen from among the radicals of formula X in which r=1 of formula Xc, as defined below: 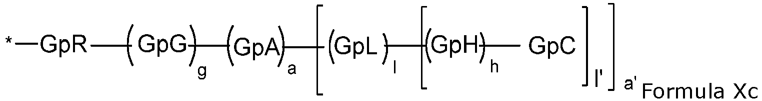 in which GpR is a radical from formula VII. 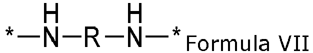 and GpG, GpA, GpL, GpH, GpC, R, a, a', g, h, I, a' and I' have the definitions given above.

In one embodiment, said at least one hydrophobic radical -Hy is chosen from among the radicals of formula X in which r=1 of formula Xc, as defined below: 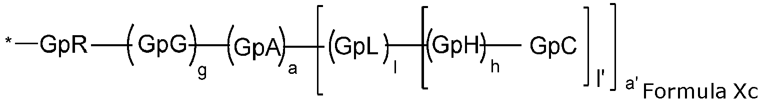 in which GpR is a radical from formula VII'. 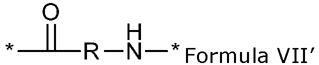 and GpG, GpA, GpL, GpH, GpC, R, a, a', g, h, I, a' and I' have the definitions given above.

In one embodiment, said at least one hydrophobic radical -Hy is chosen from among the radicals of formula X in which r=1 of formula Xc, as defined below: 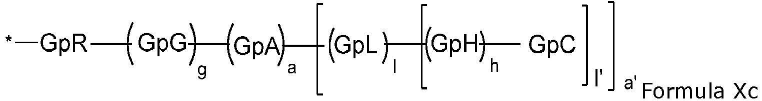 in which GpR is a radical from formula VII"

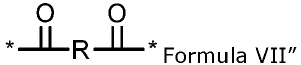

In one embodiment, r=1, and GpR is chosen from among the radicals of formulas VII' and VII" and h=0.

In one embodiment, r = 1, g = 0 and GpR is a radical of formula VII' and h=0.

In one embodiment, r =1, g = 0 and GpR is a radical of formula VII' and h=1.

In one embodiment, r=1, g = 0 and GpR is a radical of formula VII', GpA is chosen from among the radicals of Formula VIIIa or VIIIb and h=0.

In one embodiment, r=1, g = 0, GpR is a radical of formulas =VII', GpA is chosen from among the radicals of formula VIIIa or VIIIb and h = 1.

In one embodiment, r = 1, g = 0 GpR is a radical of formula VII', GpA is a radical from formula VIIIa and h=0.

In one embodiment, r =1, g = 0 GpR is a radical from formula VII', GpA is a radical of formula VIIIa and h=1.

In one embodiment, r = 1, g = 0, GpR is a radical from formula VII', GpA is a radical of formula VIIIb and h=0.

In one embodiment, r=1, g = 0, GpR is a radical of formulas =VII', GpA is a radical of formula VIIIb and h = 1.

In one embodiment, said at least one hydrophobic radical -Hy is chosen from among the radicals of formula X, as defined below: 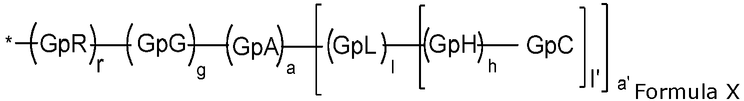 in which GpC is a radical from formula IX in which e = 0 and GpC is a radical from formula IXa.

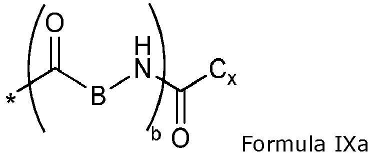

In one embodiment, said at least one hydrophobic radical -Hy is chosen from among the radicals of formula X, as defined below: 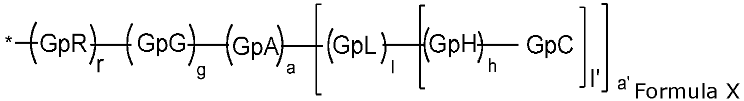 in which GpC is a radical from formula IX in which e = 1, b = 0 and GpC is a radical from formula IXd.

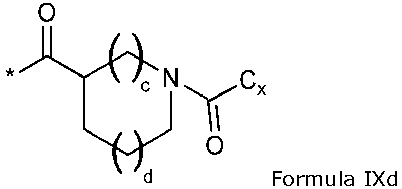

In one embodiment, said at least one hydrophobic radical -Hy is chosen from among the radicals of formula X, as defined below: 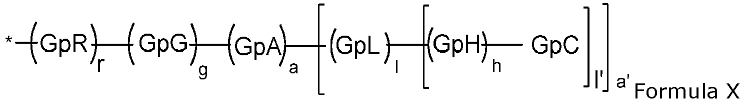 in which GpC is a radical from formula IX in which e = 1 and GpC is a radical from formula IXb

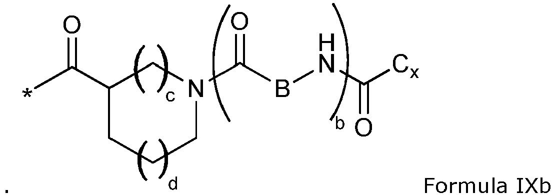

In one embodiment, said at least one hydrophobic radical -Hy is chosen from among the radicals of formula X in which r, g, a, I, h are equal to 0 of formula Xd, as defined below:

*-GpC Formula Xd,

in which GpC is a radical from formula IX in which e = 0, b = 0 and GpC is a radical from formula IXc.

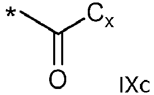

In one embodiment, the composition according to the invention is characterized in that said hydrophobic radicals are chosen from among the hydrophobic radicals of formula X in which a'=2 and a = 1 and I=0, represented by the following formula Xf: 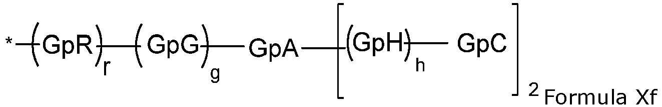 GpR, GpG, GpA, GpH, GpC, r, g and h have the definitions given above.

In one embodiment, the composition according to the invention is characterized in that said hydrophobic radicals are chosen from among the hydrophobic radicals of formula X in which h=0, I=0 and I'=1, represented by the following formula Xg: 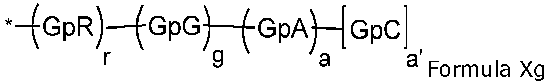 GpR, GpG, GpA, GpC, r, g, a, and a' have the definitions given above.

In one embodiment, the composition according to the invention is characterized in that said hydrophobic radicals are chosen from among the hydrophobic radicals of formula X in which h=0, a' = 1 represented by the following formula Xh: 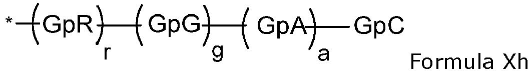 GpR, GpG, GpA, GpC, r, a and g have the definitions given above.

In one embodiment, the composition according to the invention is characterized in that said hydrophobic radicals are chosen from among the hydrophobic radicals of formula X in which h = 0, a' = 2 and a = 1, represented by the following formula Xi: 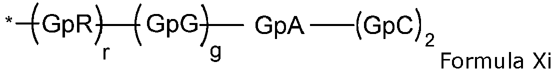 GpR, GpG, GpA, GpC, r and g have the definitions given above.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 12 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 4 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 4 atoms of carbon.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 2 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 1 to 11 atoms of carbon.

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 1 to 6 atoms of carbon.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 2 to 5 atoms of carbon and bearing one or more amide functions (-CONH2).

In one embodiment, the composition is characterized in that the hydrophobic radical from formulas X, Xa, Xb, Xb', Xc, Xe, Xf, Xg, Xh and Xi is a radical in which R is a divalent, linear alkyl radical comprising 2 to 5 atoms of carbon and bearing one or more amide functions (-CONH2).

In one embodiment, the composition is characterized in that the hydrophobic radical from formulas X, Xa, Xb, Xb', Xc, Xe, Xf, Xg, Xh and Xi is a radical in which R is chosen from the group constituted by the radicals represented by the formulas below:

| | |
|---|---|
| 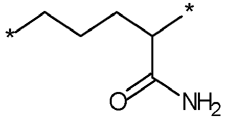 | Formula X1 |
| 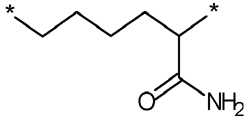 | Formula X2 |

In one embodiment, the composition is characterized in that the hydrophobic radical from formulas X, Xa, Xb, Xb', Xc, Xe, Xf, Xg, Xh and Xi is a radical in which R is a radical from Formula X1.

In one embodiment, the composition is characterized in that the hydrophobic radical from formulas X, Xa, Xb, Xb', Xc, Xe, Xf, Xg, Xh and Xi is a radical in which R is a radical from Formula X2.

In one embodiment, the composition is characterized in that the hydrophobic radical from formulas X, Xa, Xb, Xb', Xc, Xe, Xf, Xg, Xh and Xi is a radical in which R is bonded to the co-polyamino acid via an amide function borne by the carbon in delta or epsilon position (or in position 4 or 5) in relation to the amide function (-CONH₂).

In one embodiment, the composition is characterized in that the hydrophobic radical from formulas X, Xa, Xb, Xb', Xc, Xe, Xf, Xg, Xh and Xi is a radical in which R is an unsubstituted linear ether or polyether radical comprising 4 to 14 carbon atoms and from 1 to 5 oxygen atoms.

In one embodiment, the composition is characterized in that the hydrophobic radical from formulas X, Xa, Xb, Xb', Xc, Xe, Xf, Xg, Xh and Xi is a radical in which R is an ether radical.

In one embodiment, the composition is characterized in that the hydrophobic radical from formulas X, Xa, Xb, Xb', Xc, Xe, Xf, Xg, Xh and Xi is a radical in which R is an ether radical comprising 4 to 6 carbon atoms.

In one embodiment, the composition is characterized in that the hydrophobic radical from formulas X, Xa, Xb, Xb', Xc, Xe, Xf, Xg, Xh and Xi is a radical in which R is a divalent alkyl radical comprising 6 atoms of carbon.

In one embodiment, the composition is characterized in that the hydrophobic radical from formulas X, Xa, Xb, Xb', Xc, Xe, Xf, Xg, Xh and Xi is a radical in which R is an ether radical represented by formula

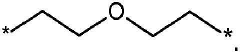

In one embodiment, the composition is characterized in that the hydrophobic radical from formulas X, Xa, Xb, Xb', Xc, Xe, Xf, Xg, Xh and Xi is a radical in which R is a polyether radical.

In one embodiment, the composition is characterized in that the hydrophobic radical from formulas X, Xa, Xb, Xb', Xc, Xe, Xf, Xg, Xh and Xi is a radical in which R is a linear polyether or polyether radical comprising 6 to 10 carbon atoms and from 2 to 3 oxygen atoms.

In one embodiment, the composition is characterized in that the hydrophobic radical from formulas X, Xa, Xb, Xb', Xc, Xe, Xf, Xg, Xh and Xi is a radical in which R is a polyether radical chosen from the group constituted by the radicals represented by the formulas below:

| | |
|---|---|
| 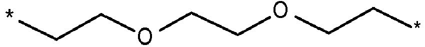 | Formula X3 |
| 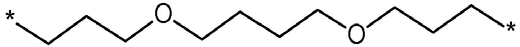 | Formula X4 |
| 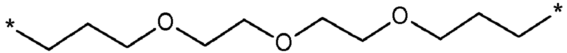 | Formula X5 |
| 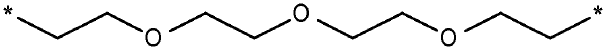 | Formula X6 |

In one embodiment, the composition is characterized in that the hydrophobic radical from formulas X, Xa, Xb, Xb', Xc, Xe, Xf, Xg, Xh and Xi is a radical in which R is a radical from Formula X3.

In one embodiment, the composition is characterized in that the hydrophobic radical from formulas X, Xa, Xb, Xb', Xc, Xe, Xf, Xg, Xh and Xi is a radical in which R is a radical from Formula X4.

In one embodiment, the composition is characterized in that the hydrophobic radical from formulas X, Xa, Xb, Xb', Xc, Xe, Xf, Xg, Xh and Xi is a radical in which R is a polyether radical chosen from the group constituted by the radicals represented by the formulas X5 and X6 below:

In one embodiment, the composition is characterized in that the hydrophobic radical from formulas X, Xa, Xb, Xb', Xc, Xe, Xf, Xg, Xh and Xi is a radical in which R is a polyether radical from Formula X5.

In one embodiment, the composition is characterized in that the hydrophobic radical from formulas X, Xa, Xb, Xb', Xc, Xe, Xf, Xg, Xh and Xi is a radical in which R is a polyether radical from Formula X6.

| | |
|---|---|
| 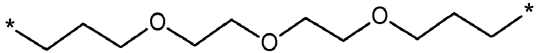 | Formula X5 |
| 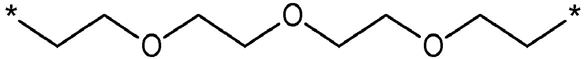 | Formula X6 |

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI' in which G is an alkyl radical comprising 6 carbon atoms represented by formula Z below:

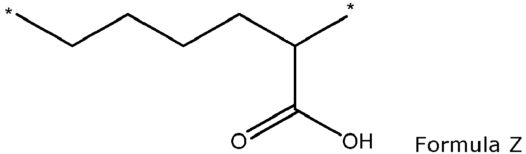

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by formula Z below:

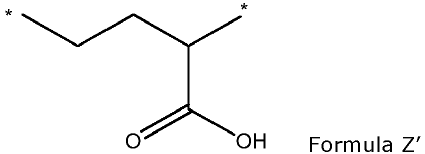

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by -(CH₂)₂-CH(COOH)-.

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 4 carbon atoms represented by -CH((CH₂)₂COOH)-.

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 3 carbon atoms represented by -CH₂-CH-(COOH).

In one embodiment, the composition is characterized in that the hydrophobic radical from formulas X, Xa, Xb, Xb', Xc, Xe, Xf, Xg, Xh and Xi is a radical in which the radical GpG and/or GpH is from Formula XI which G is an alkyl radical comprising 3 carbon atoms represented-CH(CH₂)COOH)-.

In one embodiment, the composition is characterized in that the hydrophobic radical from formulas X, Xa, Xb, Xb', Xc, Xe, Xf, Xg, Xh and Xi is a radical in which GpA is from formula VIII and in which A₁, A₂ or A₃ is chosen from the group constituted by the radicals represented by the formulas below:

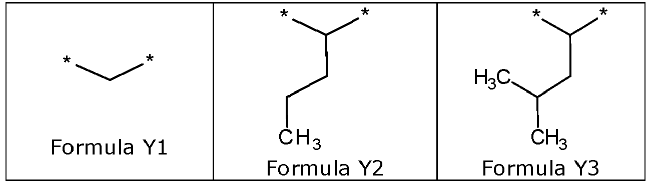

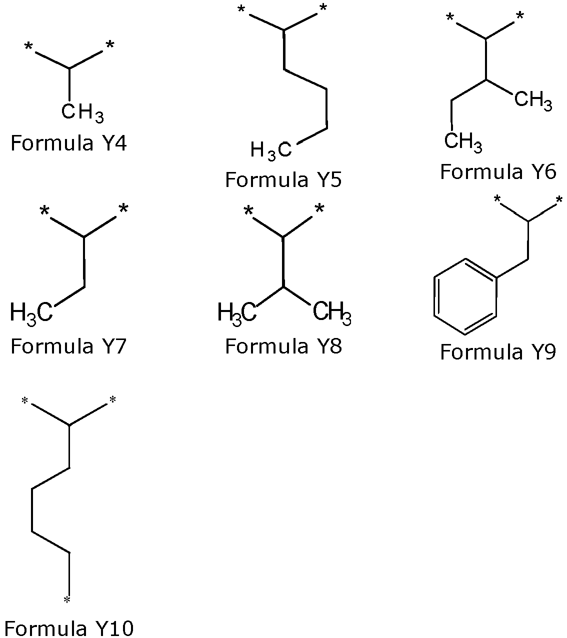

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from the group constituted by the radicals from formulas IXe, IXf or IXg represented below:

| | |
|---|---|
| 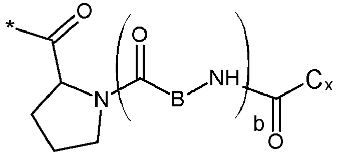 | Formula IXe |
| 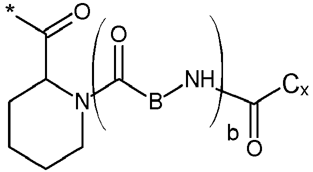 | Formula IXf |
| 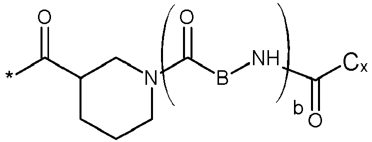 | Formula IXg |

In one embodiment, the composition is characterized in that the hydrophobic radical from formulas X, Xa, Xb, Xb', Xd,Xc, Xd, Xe, Xf, Xg, Xh and Xi is a radical in which the radical GpC from formula IX is chosen from the group constituted by the radicals from formulas IXe, IXf or IXg in which b is equal to 0, respectively responding to formulas IXh, IXi, and IXj below:

| | |
|---|---|
| 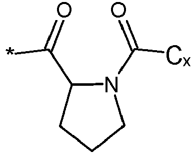 | Formula IXh |
| 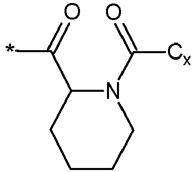 | Formula IXi |
| 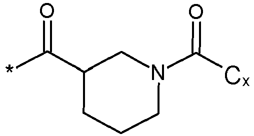 | Formula IXj |

In one embodiment, the composition is characterized in that the hydrophobic radical from formulas X, Xa, Xb, Xb', Xc, Xd, Xe, Xf, Xg, Xh and Xi is a radical in which the radical GpC corresponds to the formula IX or IXe in which b = 0, and corresponds to the formula IXh.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by the linear alkyl radicals.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by branched alkyl radicals.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 19 and 14 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the radicals represented by the formulas below:

| | |
|---|---|
| 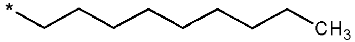 | x = 9 |
| 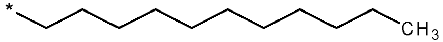 | x = 11 |
| 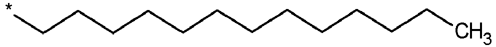 | x = 13 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 15 and 16 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the radicals represented by the formulas below:

| | |
|---|---|
| 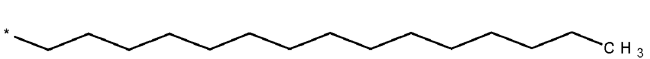 | x = 15 |

In one embodiment, the composition is characterized in that the hydrophobic radical from formulas X, Xa, Xb, Xb', Xc, Xd, Xe, Xf, Xg, Xh and Xi is a radical in which Cx is a radical is chosen from the group constituted by the radicals represented by the formulas below:

| | |
|---|---|
| 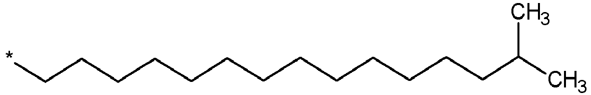 | x = 16 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 17 and 25 carbon atoms.

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 17 and 18 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical is chosen from the group constituted by the alkyl radicals represented by the formulas below:

| | |
|---|---|
| 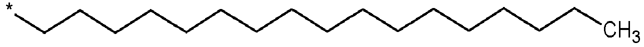 | x = 17 |

In one embodiment, the composition is characterized in that the hydrophobic radical from formulas X, Xa, Xb, Xb', Xc, Xd, Xe, Xf, Xg, Xh and Xi is a radical in which Cx is chosen from the group constituted by alkyl radicals comprising between 18 and 25 carbon atoms.

In one embodiment, the composition is characterized in that the hydrophobic radical from formulas X, Xa, Xb, Xb', Xc, Xd, Xe, Xf, Xg, Xh and Xi is a radical in which Cx is a radical chosen from the group constituted by the alkyl radicals represented by the formulas below:

| | |
|---|---|
| 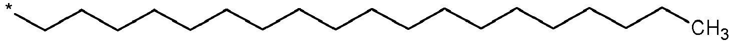 | x = 19 |
| 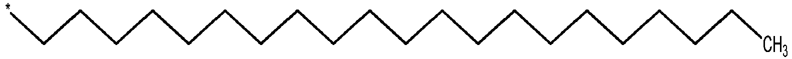 | x = 21 |

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from among the group constituted of radicals in which Cx is chosen from the group constituted by alkyl radicals comprising between 14 and 15 carbon atoms.

In one embodiment, the composition is characterized in that the hydrophobic radical from formulas X, Xa, Xb, Xb', Xc, Xd, Xe, Xf, Xg, Xh and Xi is a radical in which the radical GpC from formula IX is chosen from the group constituted of radicals in which Cx is chosen from the group constituted by the radicals represented by the formulas below:

| | |
|---|---|
| 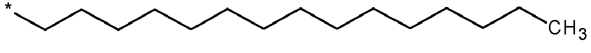 | x = 15 |

In one embodiment, when a' = 1, x is between 11 and 25 (11 ≤ x ≤ 25). In particular, when x is between 15 and 16 (x = 15 or 16) then r = 1 and R is an ether or polyether radical and when x is greater than 17 (x ≥ 17) then r = 1 and R is an ether or polyether radical.

In one embodiment, when a' = 2, x is between 9 and 15 (9 ≤ x ≤ 15).

In one embodiment, the co-polyamino acid is chosen from among the co-polyamino acids of formula XXXb in which the hydrophobic radical -Hy is chosen from the group of hydrophobic radicals from X, Xc', Xa, Xb', Xc, Xe, Xg and Xh in which a' = 1 and I' = 1 and GpC is a radical from formula IXe.

In one embodiment, the co-polyamino acid is chosen from among the co-polyamino acids of formula XXXb in which the hydrophobic radical -Hy is chosen from the group of hydrophobic radicals from formulas X, Xc', Xa, Xb', Xc, Xe, Xg and Xh in which a' = 1 and I' = 1 and GpC is a radical from formula IX in which e=0.

In one embodiment, the co-polyamino acid is chosen from among the co-polyamino acids of formula XXXb in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IXe.

In one embodiment, the co-polyamino acid is chosen from among the co-polyamino acids of formula XXXb in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IX in which e = 0.

In one embodiment, the co-polyamino acid is chosen from among the co-polyamino acids of formula XXXa in which the hydrophobic radical -Hy is chosen from the group of hydrophobic radicals from formulas X, Xc', Xa, Xb', Xc, Xe, Xg and Xh in which a' = 1 and I' = 1 and GpC is a radical from formula IXe.

In one embodiment, the co-polyamino acid is chosen from among the co-polyamino acids of formula XXXa in which the hydrophobic radical -Hy is chosen from the group of hydrophobic radicals from formulas X, Xc', Xa, Xb, Xc, Xf, Xg and Xi in which a' = 2 and I' = 2 and GpC is a radical from formula IXe.

In one embodiment, the hydrophobic radical Hy is chosen from the group of hydrophobic radicals from formula X, in which h is greater than or equal to 2 h ≥ 2) and GpC is from formula IXe.

In one embodiment, the hydrophobic radical Hy is chosen from the group of hydrophobics from formula X in which g is greater than or equal to 2 (g ≥ 2) and a, I and h are equal to 0 and GpC is from formula IXe.

In one embodiment, the composition is characterized in that the hydrophobic radical from the hydrophobic radicals from formulas X, Xc', Xa, Xb, Xb', Xc, Xe, Xg and Xh in which a' = 1 and I' = 1 and in which Cx is chosen from the group constituted by the linear alkyl radicals.

In one embodiment, the composition is characterized in that the hydrophobic radical is chosen from the hydrophobic radicals from formulas X, Xc', Xa, Xb, Xb', Xc, Xf, Xg and Xi in which a' = 2 or I' = 2 and in which Cx is chosen from the group constituted by the linear alkyl radicals.

In one embodiment, the hydrophobic radical -Hy is chosen from the group of hydrophobic radicals from formula X in which GpR is a radical from formula VII, GpH is a radical from XI and GpC is a radical from formula IX in which e = 1, b = 0 and x = 13.

In a preferred embodiment, hydrophobic radical -Hy is chosen from the group of hydrophobic radicals from formula X 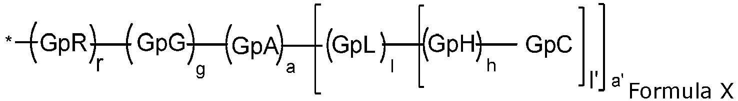 in which:
- r is an integer equal to 0 or to 1, in particular r = 1, and GpR is a radical according to Formula VII 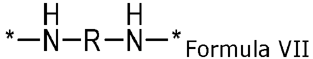
- g = 0,
- a = 0
- a' = 1
- I = 1 and GpL is a radical according to formula XII: 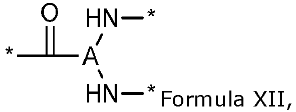
- A is a linear or branched alkyl radical and/or substituted by a radical derived from a saturated, unsaturated or aromatic ring, comprising 1 to 8 carbon atoms, in particular A is a linear or branched alkyl radical comprising 1 to 8 carbon atoms, more particularly A is a linear alkyl radical comprising 5 carbon atoms,
- I' = 2
- h = 1, 2 or 3, in particular h = 1, and GpH is a radical according to Formula XI: 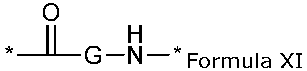
- G is a branched alkyl radical of 1 to 8 carbon atoms, with said alkyl radical bearing one or more free carboxylic acid functions, in particular one free carboxylic acid function,
- GpC is a radical according to formula IX: 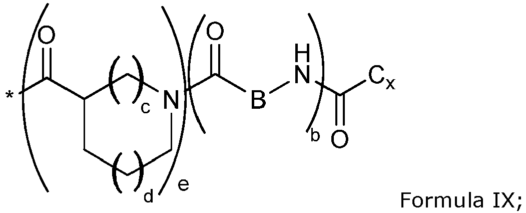 in which b = 0; e = 1; c = 0 or 1; d = 0, 1, or 2; and if c is equal to 0, then d is equal to 1 or to 2; in particular c = 0 and d = 1,
- Cₓ is a linear or branched monovalent alkyl radical, and/or comprising a cyclic part, in which x indicates the number of carbon atoms and when the hydrophobic radical -Hy carries 2 -GpC, then 9 ≤ x ≤ 15, in particular x = 13,

In an embodiment, the hydrophobic radical(s) -Hy according to formula X being bonded to the PLG:
∘ via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
∘ via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,

- the ratio M between the number of hydrophobic radicals and the number of glutamic or aspartic units being from 0 < M ≤ 0.5;
- when several hydrophobic radicals are borne by a co-polyamino acid, then they are identical or different,
- the degree of polymerization DP in glutamic or aspartic units for PLG chains is comprised from 5 to 250;
- free carboxylic acid functions being in the form of an alkali metal salt chosen from the group consisting of Na⁺ and K⁺.

In one embodiment, the composition according to the invention is characterized in that the M ratio between the number of hydrophobic radicals and the number of glutamate or aspartic unites is between 0.007 and 0.3.

In one embodiment, the composition according to the invention is characterized in that the M ratio between the number of hydrophobic radicals and the number of glutamate or aspartic unites is between 0.01 and 0.3.

In one embodiment, the composition according to the invention is characterized in that the M ratio between the number of hydrophobic radicals and the number of glutamate or aspartic unites is between 0.02 and 0.2.

In one embodiment, the composition according to the invention is characterized in that the hydrophobic radical corresponds to formula X and the M ratio between the number of hydrophobic radicals and the number of glutamate or aspartic unites is between 0.007 and 0.15.

In one embodiment, the composition according to the invention is characterized in that the hydrophobic radical corresponds to formula X and the M ratio between the number of hydrophobic radicals and the number of glutamate or aspartic unites is between 0.01 and 0.1.

In one embodiment, the composition according to the invention is characterized in that the hydrophobic radical corresponds to formula X and the M ratio between the number of hydrophobic radicals and the number of glutamate or aspartic unites is between 0.02 and 0.08.

In one embodiment, the composition according to the invention is characterized in that the hydrophobic radical corresponds to formula X in which radical Cx comprises between 9 and 10 atoms and the M ratio between the number of hydrophobic radicals and the number of glutamate or aspartic unites is between 0.03 and 0.15.

In one embodiment, the composition according to the invention is characterized in that the hydrophobic radical corresponds to formula X in which radical Cx comprises between 11 and 12 atoms and the M ratio between the number of hydrophobic radicals and the number of glutamate or aspartic unites is between 0.015 and 0.1.

In one embodiment, the composition according to the invention is characterized in that the hydrophobic radical corresponds to formula X in which radical Cx comprises between 11 and 12 atoms and the M ratio between the number of hydrophobic radicals and the number of glutamate or aspartic unites is between 0.02 and 0.08.

In one embodiment, the composition according to the invention is characterized in that the hydrophobic radical corresponds to formula X in which radical Cx comprises between 13 and 15 atoms and the M ratio between the number of hydrophobic radicals and the number of glutamate or aspartic unites is between 0.01 and 0.1.

In one embodiment, the composition according to the invention is characterized in that the hydrophobic radical corresponds to formula X in which radical Cx comprises between 13 and 15 atoms and the M ratio between the number of hydrophobic radicals and the number of glutamate or aspartic unites is between 0.01 and 0.06.

In one embodiment, the composition according to the invention is characterized in that the hydrophobic radical corresponds to formula X and the M ratio between the number of hydrophobic radicals and the number of glutamate or aspartic unites is between 0.007 and 0.3.

In one embodiment, the composition according to the invention is characterized in that the hydrophobic radical corresponds to formula X and the M ratio between the number of hydrophobic radicals and the number of glutamate or aspartic unites is between 0.01 and 0.3.

In one embodiment, the composition according to the invention is characterized in that the hydrophobic radical corresponds to formula X and the M ratio between the number of hydrophobic radicals and the number of glutamate or aspartic unites is between 0.015 and 0.2.

In one embodiment, the composition according to the invention is characterized in that the hydrophobic radical corresponds to formula X in which radical Cx comprises between 11 and 14 atoms and the M ratio between the number of hydrophobic radicals and the number of glutamate or aspartic unites is between 0.1 and 0.2.

In one embodiment, the composition according to the invention is characterized in that the hydrophobic radical corresponds to formula X in which radical Cx comprises between 15 and 16 atoms and the M ratio between the number of hydrophobic radicals and the number of glutamate or aspartic unites is between 0.04 and 0.15.

In one embodiment, the composition according to the invention is characterized in that the hydrophobic radical corresponds to formula X in which radical Cx comprises between 17 and 18 atoms and the M ratio between the number of hydrophobic radicals and the number of glutamate or aspartic unites is between 0.02 and 0.06.

In one embodiment, the composition according to the invention is characterized in that the hydrophobic radical corresponds to formula X in which radical Cx comprises between 19 and 25 atoms and the M ratio between the number of hydrophobic radicals and the number of glutamate or aspartic unites is between 0.01 and 0.06.

In one embodiment, the composition according to the invention is characterized in that the hydrophobic radical corresponds to formula X in which radical Cx comprises between 19 and 25 atoms and the M ratio between the number of hydrophobic radicals and the number of glutamate or aspartic unites is between 0.01 and 0.05.

In one embodiment, the composition according to the invention is characterized in that the co-polyamino acid bearing carboxylate charges and hydrophobic radicals is chosen from among the co-polyamino acids from formula XXXa' below: 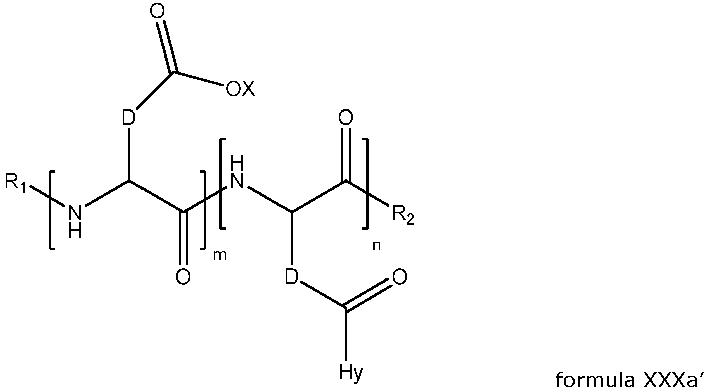 In which
- D represents, independently, either a -CH₂- group (aspartic unit) or a -CH₂-CH₂- group (glutamic unit),
- Hy is a hydrophobic radical chosen from among the hydrophobic radicals from formula X, in which r=1 and GpR is a radical from formula VII,
- R₁ is a hydrophobic radical chosen from among the hydrophobic radicals of formula X in which r=0 or r=1 and GpR is a radical from Formula VII', or a radical chosen from the group constituted by an H, a linear acyl group in C2 to C10, a branched acyl group in C4 to C10, a benzoyl, a terminal "amino acid" and a pyroglutamate,
- R₂ is a hydrophobic radical chosen from among the hydrophobic radicals of formula X in which r=1 and GpR is a radical from Formula VII, or a -NR'R" radical, with R' and R", either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetero-atoms, chosen from the group comprised of O, N and S;
- X represents a cationic entity chosen from the group comprising alkaline cations;
- the sum n + m represents the degree of polymerization DP of the co-polyamino acid, namely, the average number of monomeric units per co-polyamino acid chain and 5 ≤ n + m ≤ 250.

When the co-polyamino acid comprises one or more aspartic units, the latter may be subject to structural re-arrangements.

In one embodiment, the composition according to the invention is characterized in that when the co-polyamino acid comprises aspartic units, then the co-polyamino acid may also comprise monomeric units from formula XXXI and/or XXXI'.

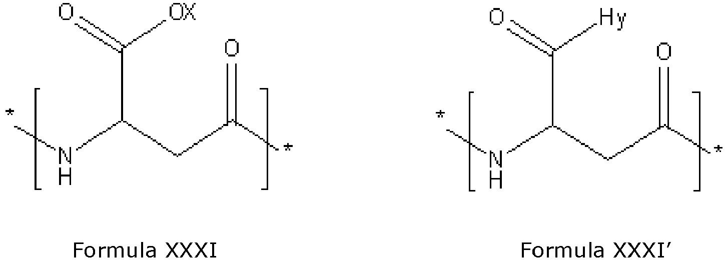

We call "a statistically grafted co-polyamino acid" a co-polyamino acid bearing carboxylate charges and at least one hydrophobic radical, a co-polyamino acid of formula XXXa.

In one embodiment, the composition according to the invention is characterized in that the co-polyamino acid bearing carboxylate charges and hydrophobic radicals is chosen from among the co-polyamino acids from formula XXXa', in which R₁ = R'₁ and R₂ = R'₂, from formula XXXa below: 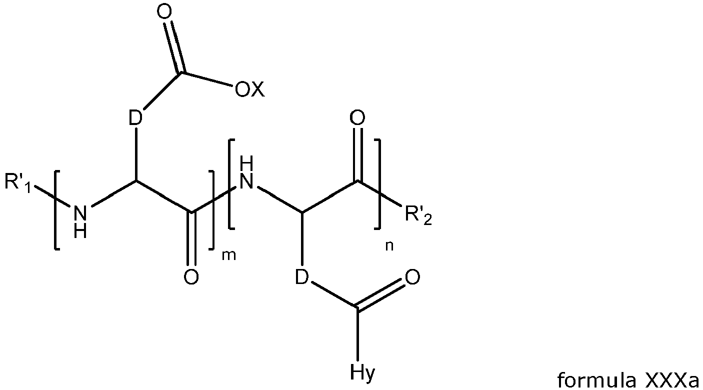 in which
- m, n, X, D and Hy have the definitions given above,
- R'₁ is a radical chosen from among the group constituted by an H, a linear acyl group in C2 to C10, a branched acyl group in C4 to C10, a benzoyl, a terminal "amino acid" and a pyroglutamate,
- R'₂ is a -NR'R" radical with R' and R" , either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetero-atoms, chosen from the group comprised of O, N and S.

In one embodiment, the composition according to the invention is characterized in that the co-polyamino acid bearing carboxylate charges and hydrophobic radicals is chosen from among the co-polyamino acids from formula XXXa in which Hy is a radical from formula X.

In one embodiment, the composition according to the invention is characterized in that the co-polyamino acid bearing carboxylate charges and hydrophobic radicals is chosen from among the co-polyamino acids from formula XXXa in which Hy is a radical from formula X, in which r=1.

In one embodiment, the composition according to the invention is characterized in that the co-polyamino acid bearing carboxylate charges and hydrophobic radicals is chosen from among the co-polyamino acids from formula XXXa, in which Hy is a radical from formula X, in which r=1, and for GpC, b=0.

In one embodiment, the composition according to the invention is characterized in that the co-polyamino acid bearing carboxylate charges and hydrophobic radicals is chosen from among the co-polyamino acids from formula XXXa in which Hy is a radical from formula X and in which GpC is a radical from formula IX.

In one embodiment, the composition according to the invention is characterized in that the co-polyamino acid bearing carboxylate charges and hydrophobic radicals is chosen from among the co-polyamino acids from formula XXXa in which Hy is a radical from formula X and in which GpC is a radical from formula IX and r=1.

We call "a semi-telechelic grafted co-polyamino acid" a co-polyamino acid bearing carboxylate charges and at least one hydrophobic radical, a co-polyamino acid of formula XXXb.

In one embodiment, the composition according to the invention is characterized in that the co-polyamino acid bearing carboxylate charges and hydrophobic radicals is chosen from among the co-polyamino acids from formula XXXa', in which n=0 from the following formula XXXb: 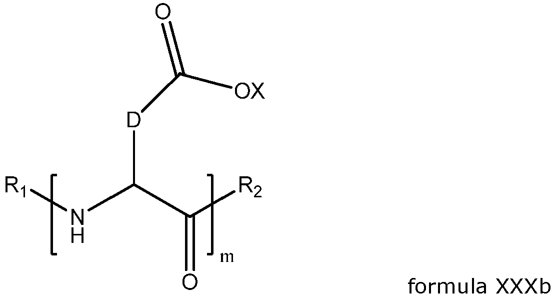 In which m, X, D, R₁ and R₂ have the definitions given above and at least R₁ or R₂ is a hydrophobic radical from formula X.

In one embodiment the composition according to the invention is characterized in that the co-polyamino acid bearing carboxylate charges and hydrophobic radicals is chosen from among the co-polyamino acids from formula XXXa' in which n=0 of formula XXXb and R₁ or R₂ is a hydrophobic radical from formula X.

In one embodiment, the composition according to the invention is characterized in that the co-polyamino acid bearing carboxylate charges and hydrophobic radicals is chosen from among the co-polyamino acids from formula XXXb in which R1= R'1 from formula XXXb': 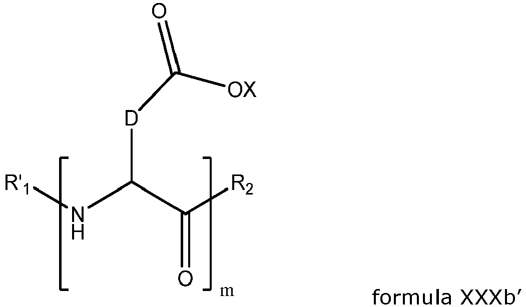 in which m, X, D, R'₁ and R₂ have the definitions given above and R₂ is a hydrophobic radical from formula X.

In one embodiment, the composition according to the invention is characterized in that the co-polyamino acid bearing carboxylate charges and hydrophobic radicals is chosen from among the co-polyamino acids from formula XXXb in which R₂ = R'₂ from formula XXXb": 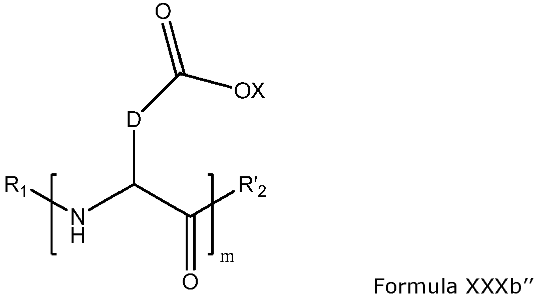 in which m, X, D, R₁ and R'₂ have the definitions given above and R₁ is a hydrophobic radical from formula X.

In a most preferred embodiment, the co-polyamino acid bearing carboxylate charges and hydrophobic radicals is according to Formula XXXb'.

In another most preferred embodiment, the co-polyamino acid bearing carboxylate charges and hydrophobic radicals is according to Formula XXXb".

In one embodiment, the composition according to the invention is characterized in that the co-polyamino acid bearing carboxylate charges and hydrophobic radicals is chosen from among the co-polyamino acids from formula XXXb or XXXb" in which R₁ is a radical from formula X and in which GpR is from formula VII'.

In one embodiment, the composition according to the invention is characterized in that the co-polyamino acid bearing carboxylate charges and hydrophobic radicals is chosen from among the co-polyamino acids from formula XXXb or XXXb" in which R₁ is a radical from formula X and in which GpR is a from formula VII".

In one embodiment, the composition according to the invention is characterized in that the co-polyamino acid bearing carboxylate charges and hydrophobic radicals is chosen from among the co-polyamino acids from formula XXXb or XXXb" in which R₁ is a hydrophobic radical from formula X and in which GpR is from formula VII' and GpC is from formula IX.

In one embodiment, the composition according to the invention is characterized in that the co-polyamino acid bearing carboxylate charges and hydrophobic radicals is chosen from among the co-polyamino acids from formula XXXb or XXXb" in which R₁ is a hydrophobic radical from formula X and in which GpR is from formula VII' and GpC is from formula IX.

In one embodiment, the composition according to the invention is characterized in that the co-polyamino acid bearing carboxylate charges and hydrophobic radicals is chosen from among the co-polyamino acids from formula XXXb or XXXb' in which R₂ is a hydrophobic radical from formula X and in which r = 1 and GpR is from formula VII.

In one embodiment, the composition according to the invention is characterized in that the co-polyamino acid bearing carboxylate charges and hydrophobic radicals is chosen from the co-polyamino acids of formula XXXa' in which at least one of R₁ or R₂ is a hydrophobic radical as defined above from the following formula XXX: 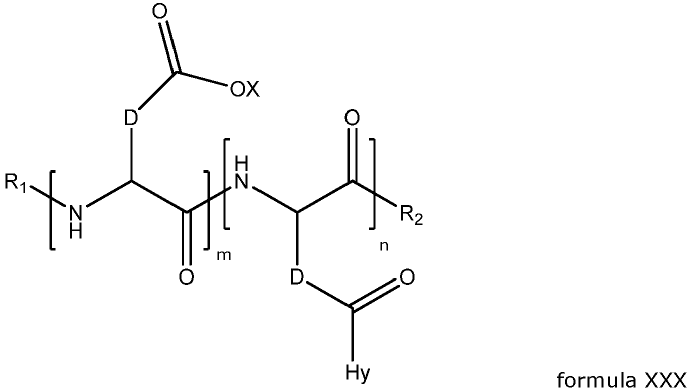 In which
- D represents, independently, either a -CH₂- group (aspartic unit) or a -CH₂-CH₂-group (glutamic unit),
- Hy is a hydrophobic radical chosen from among the hydrophobic radicals from formula X, in which r=1 and GpR is a radical from formula VII,
- R₁ is a hydrophobic radical chosen from among the hydrophobic radicals of formula X in which r=0 or r=1 and GpR is a radical from Formula VII', or a radical chosen from the group constituted by an H, a linear acyl group in C2 to C10, a branched acyl group in C4 to C10, a benzoyl, a terminal "amino acid" unit and a pyroglutamate,
- R₂ is a hydrophobic radical chosen from among the hydrophobic radicals of formula X in which r=1 and GpR is a radical from Formula VII, or a -NR'R" radical, with R' and R", either identical or different, being chosen from the group comprised by H, linear or branched or cyclic alkyls in C2 to C10, or benzyl and said R' and R" alkyls being able to form together one or more carbonated cycles, saturated, unsaturated and/or aromatic, able to comprise hetro-atoms, chosen from the group comprised of O, N and S,
- X represents an H or a cationic entity chosen from the group comprising the metallic cations;
- the sum n + m represents the degree of polymerization DP of the co-polyamino acid, namely, the average number of monomeric units per co-polyamino acid chain and 5 ≤ n + m ≤ 250.

According to an embodiment, the co-polyamino acid bearing carboxylate charges and hydrophobic radicals is as disclosed in WO2019110797 and its US equivalent US2019275115, which are incorporated by reference.

In one embodiment, the composition according to the invention is characterized in that the co-polyamino acid bearing carboxylate charges and hydrophobic radicals is chosen from the co-polyamino acids of formula A-1

**[Q(PLG)ₖ] [Hy]ⱼ[Hy]_{j'}** Formula A-I

Wherein:
- j ≥ 1; 0 ≤ j' ≤ n'1 and j + j' ≥ 1 and k ≥ 2
- said co-polyamino acid according to formula A-I bearing at least one hydrophobic radical -Hy, carboxylate charges and consisting of at least two chains of glutamic or aspartic units PLG bound together by at least a divalent linear or branched radical or spacer Q[-*]ₖconsisting of an alkyl chain comprising one or a plurality of heteroatoms chosen in the group consisting of nitrogen and oxygen atoms and/or bearing one or a plurality of heteroatoms consisting of nitrogen and oxygen radicals and/or radicals bearing one or a plurality of heteroatoms consisting of nitrogen and oxygen atoms and/or carboxyl functions,
   - said radical or spacer Q[-*]ₖ being bound to at least two glutamic or aspartic unit chains PLG by an amide function and,
   - said amide functions binding said radical or spacer Q[-*]ₖ bound to said at least two chains of glutamic or aspartic units result from the reaction between an amine function and an acid function respectively borne either by the precursor Q' of the radical or spacer Q[-*]ₖ or by a glutamic or aspartic unit,
   - said hydrophobic radical -Hy being bound either to a terminal "amino acid" unit and then j ≥ 1, or to a carboxyl function borne by one of the chains of the glutamic or aspartic units PLG and then j' = n'1 and n'1 is the mean number of monomeric units bearing a hydrophobic radical -Hy.

In one embodiment, k is 2, 3, 4, 5 or 6.

In one embodiment, k = 2.

In one embodiment, k = 3.

In one embodiment, k = 4.

In one embodiment, k = 5.

In one embodiment, k = 6.

In one embodiment, j is 1, 2, 3, 4, 5 or 6.

In one embodiment, j = 1.

In one embodiment, j = 2.

In one embodiment, j = 3.

In one embodiment, j = 4.

In one embodiment, j = 5.

In one embodiment, j = 6.

In one embodiment, the radical or spacer Q[-*]ₖ is represented by a radical according to formula A-II:

**Q[-*]ₖ = ([Q']q)[-*]k** Formula A-II

Wherein 1≤ q ≤ 5
- The radicals Q' being identical or different and chosen in the group consisting of radicals of the following formulas III to VI', to form Q[-*]ₖ:
   by a radical according to formula A-III 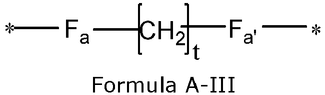 wherein 1 ≤ t ≤ 8
   by a radical according to formula A-IV: 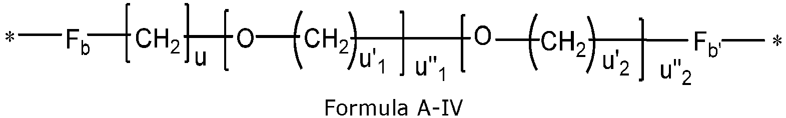 wherein:
      At least one of u₁" or u₂" is different to 0.
      If u₁" ≠ 0 then u₁' ≠ 0 and if u₂" ≠ 0 then u₂' ≠ 0,
      u₁' and u₂' are identical or different and,
      2 ≤ u ≤ 4,
      0 ≤ u₁" ≤ 8, in particular 0 ≤ u₁" ≤ 4,
      0 ≤ u₂' ≤ 4
      0 ≤ u₂" ≤ 8, in particular 0 ≤ u₂" ≤ 4,
   by a radical according to formula A-V: 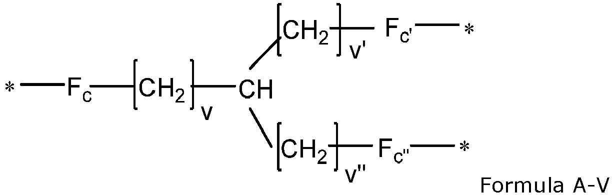 Wherein:
      v, v' and v" identical or different, are integers ≥ 0, and
      v + v' +v" ≤ 15,
   by a radical according to formula A-VI: 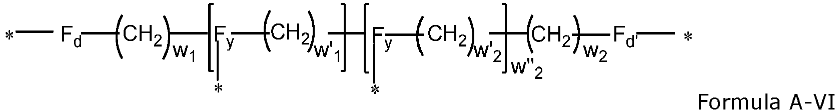
   Wherein:
   w₁' is different to 0,
   0 ≤ w₂" ≤ 1,
   w₁ ≤ 6 and w₁'≤ 6 and/or w₂ ≤ 6 and w₂'≤ 6
   where Fd, and Fd' identical or different representing functions -NH- or -CO- and Fy representing a trivalent nitrogen atom -N=,
   where in each of the radicals represented above Fx = Fa, Fb, Fc, Fd, Fa', Fb', Fc', Fc" and Fd' identical or different representing functions -NH- or -CO- and Fy representing a trivalent nitrogen atom -N=,
   two radicals Q' being bound together by a covalent bond between a carbonyl function, Fx = -CO-, and an amine function Fx = -NH- or Fy = -N= , thus forming an amide bond and where a function Fx = Fa, Fb, Fc, Fd, Fa', Fb', Fc', Fc" and Fd' is not used in a bond between two Q', this function is then free and salified.

In one embodiment, said radical Q' is chosen among the radicals according to formula A-VI', wherein w₂ = 0 according to formula VI' as defined hereinafter: 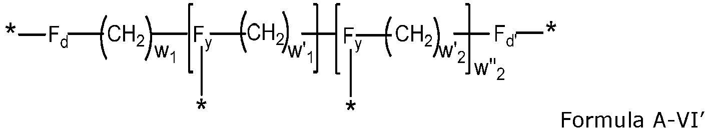 wherein:
w'₁ is different to 0,
0 ≤ w"₂ ≤ 1,
w₁ ≤ 6 and w'₁≤ 6 and/or w'₂≤ 6
where Fd, and Fd' identical or different representing functions -NH- or -CO- and Fy representing a trivalent nitrogen atom -N=,
two radicals Q' being bound together by a covalent bond between a carbonyl function, Fx = -CO-, and an amine function Fx = -NH- or Fy = -N= , thus forming an amide bond,
where in each of the radicals represented above, Fx = Fa, Fb, Fc, Fd, Fa', Fb', Fc', Fc" and Fd' identical or different representing functions -NH- or -CO- and Fy representing a trivalent nitrogen atom -N=,
two radicals Q' being bound together by a covalent bond between a carbonyl function, Fx = -CO-, and an amine function Fx = -NH- or Fy = -N= , thus forming an amide bond.

In one embodiment, if Fa and Fa' are -NH-, then t≥2.

In one embodiment, if Fa and Fa' are -CO-, then t≥1.

In one embodiment, if Fa and Fa' are -CO- and -NH-, then t≥1.

In one embodiment, if Fb and Fb' are -NH-, then u and u₁'≥2 and/or u₂'≥2.

In one embodiment, if Fc, Fc' and Fc" are -NH- then at least two of v, v' and v" are different to 0.

In one embodiment, if Fc, Fc' and Fc" are 2 -NH- and 1 -CO- then at least one of the indices of the -(CH₂)- bearing a nitrogen is different to 0.

In one embodiment, if Fc, Fc' and Fc" are 1 -NH- and 2 -CO- then no conditions.

In one embodiment, if Fc, Fc' and Fc" are -CO- then at least one of v, v' and v" is different to 0.

In one embodiment, if Fd and Fd' are -NH-, w1 and w1' ≥2 and/or w2 and w'2 ≥2.

In one embodiment, if Fd and Fd' are -CO-, w1 and w1' ≥1 and/or w2 and w2' ≥1.

In one embodiment, if Fd and Fd' are -CO- and -NH-, w1 and w1' ≥1 and/or w2 and w2' ≥ 1.

The at least two chains of glutamic or aspartic units PLG being bound to Q[-*]ₖ by a function Fx or Fy by a covalent bond to form an amide bond with a function -NH- or -CO- of the PLG.

In one embodiment, 1≤ q ≤ 5.

In one embodiment, v + v' +v" ≤ 15.

In one embodiment, at least one of the Q' is a radical according to formula A-III, 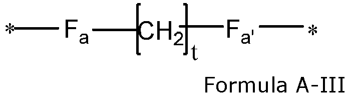 wherein the precursor is a diamine.

In one embodiment, the precursor of the radical according to formula A-III is a diamine chosen in the group consisting of ethylene diamine, butylenediamine, hexylenediamine, 1,3-diaminopropane and 1,5-diaminopentane, propylene diamine, pentylene diamine.

In one embodiment, t = 2 and the precursor of the radical according to formula A-III is ethylenediamine.

In one embodiment, t = 4 and the precursor of the radical according to formula A-III is butylenediamine.

In one embodiment, t = 6 and the precursor of the radical according to formula A-III is hexylenediamine.

In one embodiment, t = 3 and the precursor of the radical according to formula A-III is 1,3-diaminopropane.

In one embodiment, t = 5 and the precursor of the radical according to formula A-III is 1,5-diaminopentane.

In one embodiment, the precursor of the radical according to formula A-III is an amino acid.

In one embodiment, the precursor of the radical according to formula A-III is an amino acid chosen in the group consisting of aminobutanoic acid, aminohexanoic acid and beta-alanine.

In one embodiment, t = 2 and the precursor of the radical according to formula A-III is beta-alanine.

In one embodiment, t = 6 and the precursor of the radical according to formula A-III is aminohexanoic acid.

In one embodiment, t = 4 and the precursor of the radical according to formula A-III is aminobutanoic acid

In one embodiment, the precursor of the radical according to formula A-III is a diacid.

In one embodiment, the precursor of the radical according to formula A-III is a diacid chosen in the group consisting of succinic acid, glutaric acid and adipic acid.

In one embodiment, t = 2 and the precursor of the radical according to formula A-III is succinic acid.

In one embodiment, t = 3 and the precursor of the radical according to formula A-III is glutaric acid.

In one embodiment, t = 4 and the precursor of the radical according to formula A-III is adipic acid.

In one embodiment, at least one of the Q' is a radical according to formula A-IV, 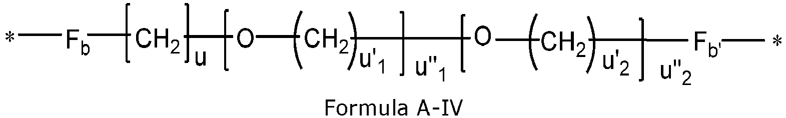 wherein the precursor is a diamine.

In one embodiment, the precursor of the radical according to formula A-IV is a diamine chosen in the group consisting of diethyleneglycoldiamine, triethyleneglycol diamine, 1-amino-4,9-dioxa-12-dodecanamine and 1-amino-4,7,10-trioxa-13-tridecanamine.

In one embodiment, u = u'₁ = 2, u"₁=1, u"₂ = 0 and the precursor of the radical according to formula A-IV is diethyleneglycol diamine.

In one embodiment, u = u'₁ = u'₂ = 2, u"₁= u"₂ = 1 and the precursor of the radical according to formula A-IV is triethyleneglycol diamine.

In one embodiment, u = u'₂ = 3, u'₁ = 4, u"₁= u"₂ = 1 and the precursor of the radical according to formula A-IV is 4,9-dioxa-1,12-dodecanediamine.

In one embodiment, u = u'₂ = 3, u'₁ = u"₁= 2, u"₂ = 1 and the precursor of the radical according to formula A-IV is 4,7,10-trioxa-1,13-tridecanediamine.

In one embodiment, at least one of the Q' is a radical according to formula A-V, 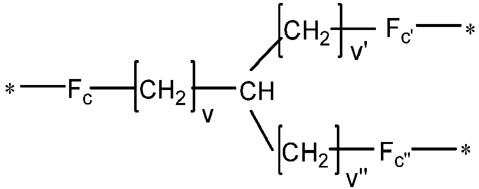 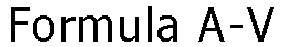 wherein the precursor is chosen in the group consisting of amino acids.

In one embodiment, the precursor of the radical according to formula A-V is an amino acid chosen in the group consisting of lysine, ornithine, 1,3-diaminopropionic acid.

In one embodiment, v = 4, v' = v" = 0 and the precursor of the radical according to formula A-V is lysine.

In one embodiment, v = 3, v' = v" = 0 and the precursor of the radical according to formula A-V is ornithine.

In one embodiment, v = 2, v' = v" = 0 and the precursor of the radical according to formula A-V is 2,3-diaminopropionic acid.

In one embodiment, at least one of the Q' is a radical according to formula A-V, 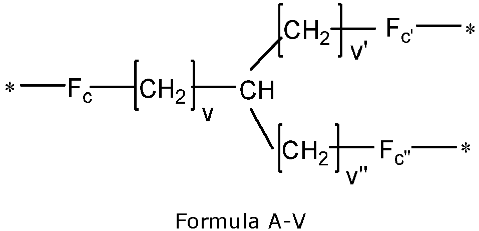 wherein the precursor is chosen in the group consisting of triacids.

In one embodiment, the precursor of the radical according to formula A-V is a triacid chosen in the group consisting of tricarballylic acid.

In one embodiment, v = 0, v' = v" = 1 and the precursor of the radical according to formula A-V is tricarballylic acid.

In one embodiment, at least one of the Q' is a radical according to formula A-V, 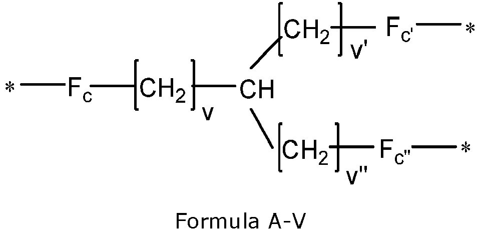 wherein the precursor is chosen in the group consisting of triamines.

In one embodiment, the precursor of the radical according to formula A-V is a triamine chosen in the group consisting of (2-(aminomethyl)propane-1,3-diamine).

In one embodiment, v = v' = v" = 1 and the precursor of the radical according to formula A-V is (2-(aminomethyl)propane-1,3-diamine).

In one embodiment, at least one of the Q' is a radical according to formula A-VI, 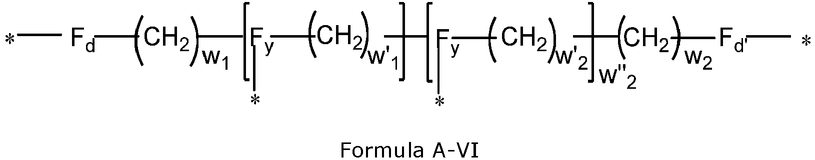 wherein the precursor is a triamine.

In one embodiment, w"₂ = 0 and the precursor of the radical according to formula A-VI is a triamine chosen in the group consisting of spermidine, norspermidine, and diethylenetriamine and bis(hexamethylene)triamine.

In one embodiment, w"₂ = 0 and the precursor of the radical according to formula A-VI is spermidine.

In one embodiment, w"₂ = 0 and the precursor of the radical according to formula A-VI is norspermidine.

In one embodiment, w"₂ = 0 and the precursor of the radical according to formula A-VI is diethylenetriamine.

one embodiment, w"₂ = 0 and the precursor of the radical according to formula A-VI is bis(hexamethylene)triamine.

In one embodiment, at least one of the Q' is a radical according to formula A-VI, 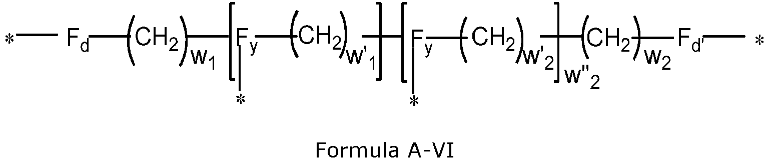 wherein the precursor is a tetramine.

In one embodiment, w"₂ = 1 and the precursor of the radical according to formula A-VI is a tetramine.

In one embodiment, w"₂ = 1 and the precursor of the radical according to formula A-VI is a tetramine chosen in the group consisting of spermine and triethylenetetramine.

In one embodiment, w"₂ = 1 and the precursor of the radical according to formula A-VI is spermine.

In one embodiment, w"₂ = 1 and the precursor of the radical according to formula A-VI is triethylenetetramine.

In one embodiment, the precursor of the radical or spacer Q[-*]ₖ has 4 reactive functions, chosen among the amine and carboxylic acid functions. Such a precursor may be 1,2,3,4-butanetetraoic acid.

In one embodiment, at least one of the Q' is a radical according to formula A-VI', 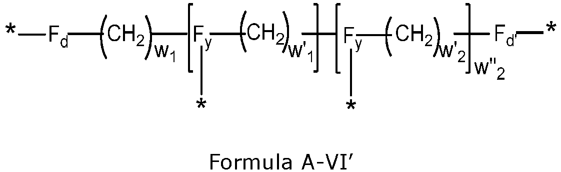 wherein the precursor is a triamine.

In one embodiment, w"2 = 0 and the precursor of the radical according to formula A-VI' is a triamine chosen in the group consisting of spermidine, norspermidine, and diethylenetriamine and bis(hexamethylene)triamine.

In one embodiment, w"2 = 0 and the precursor of the radical according to formula A-VI' is spermidine.

In one embodiment, w"2 = 0 and the precursor of the radical according to formula A-VI' is norspermidine.

In one embodiment, w"2 = 0 and the precursor of the radical according to formula A-VI' is diethylenetriamine.

one embodiment, w"2 = 0 and the precursor of the radical according to formula A-VI is bis(hexamethylene)triamine.

In one embodiment, at least one of the Q' is a radical according to formula A-VI', 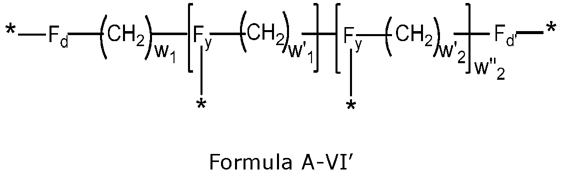 wherein the precursor is a tetramine.

In one embodiment, w"2 = 1 and the precursor of the radical according to formula A-VI' is a tetramine.

In one embodiment, w"2 = 1 and the precursor of the radical according to formula A-VI' is a tetramine chosen in the group consisting of spermine and triethylenetetramine.

In one embodiment, w"2 = 1 and the precursor of the radical according to formula A-VI' is spermine.

In one embodiment, w"2 = 1 and the precursor of the radical according to formula A-VI' is triethylenetetramine.

In one embodiment, the precursor of the radical or spacer Q[-*]ₖ has 4 reactive functions, chosen among the amine and carboxylic acid functions.

In one embodiment, the precursor of the radical or spacer Q[-*]ₖ has 4 reactive functions and the precursor of the radical or spacer Q[-*]ₖ is 1,2,3,4-butanetetraoic acid.

In one embodiment, all the Fx are bound to the PLG or to other Fx or Fy.

In one embodiment, one or plurality of Fx are free, i.e. are not bound to the PLG, or to another Fx, or to an Fy.

In one embodiment, one Fx is free, i.e. is not bound to the PLG, or to another Fx, or to an Fy.

In one embodiment, the -CO- type Fx(s) is free, it is in carboxylic acid salt form.

In one embodiment, the free -CO- type Fx is borne by a radical Q' according to Formula A-V.

In one embodiment, the -NH- type Fx(s) is free, it is in amine or ammonium form.

In one embodiment, the PLG are bound to Fx where Fx = -NH- or to Fy by at least one carbonyl function of the PLG.

In one embodiment, the PLG are bound to Fx where Fx = -NH- or to Fy by at least one carbonyl function which is not in the C-terminal position of the PLG.

In one embodiment, the PLG are bound to Fx where Fx = -NH- or to Fy by the carbonyl function in the C-terminal function of the PLG.

In one embodiment, the PLG are bound to Fx where Fx = -NH- by the carbonyl function in the C-terminal function of the PLG.

In one embodiment, the PLG are bound to Fx where Fx = Fy by the carbonyl function in the C-terminal function of the PLG.

In one embodiment, the PLG are bound to Fx, where Fx = -CO- by the nitrogen atom in the N-terminal function of the PLG.

In a preferred embodiment, Q' is either a radical of formula A-III or A-IV 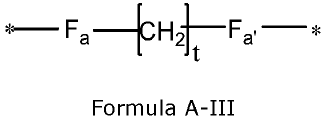 wherein 1 ≤ t ≤ 8, in particular with Fa and Fa' being -NH- and 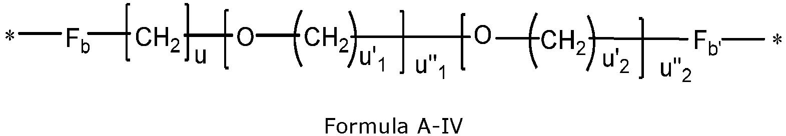 wherein:
at least one of u₁" or u₂" is different to 0.
If u₁" ≠ 0 then u₁' ≠ 0 and if u₂" ≠ 0 then u₂' ≠ 0,
u₁' and u₂' are identical or different and,
2 ≤ u ≤ 4,
0 ≤ u₁' ≤ 4,
0 ≤ u₁" ≤ 8, in particular 0 ≤ u₁" ≤ 4,
0 ≤ u₂' ≤ 4
0 ≤ u₂" ≤ 8, in particular 0 ≤ u₂" ≤ 4,
and Fa, Fb, Fa', Fb' identical or different are representing functions -NH- or -CO-. They would form an amide bond with the aspartate or glutamate unit of the PLG they are liked with.

In a most preferred embodiment, Q' is a radical of formula A-III 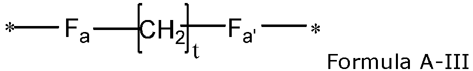 Wherein
- t is 2, 3 or 4, in particular 2 and
- Fa, and Fa' identical or different are representing functions -NH- or -CO-, in particular Fa = Fa' and represents -NH- They would form an amide bond with the aspartate or glutamate unit of the PLG they are liked with.

In a most preferred embodiment, Q' is a radical of formula A-IV wherein u is 2, u'1 and u'2 are equal to 2, u"1 is an integer comprised between 0 and 8 and u"2 is an integer comprised between 0 and 8 and thus corresponds to Formula A-IVa

*-Fb-(CH₂)₂-(O(CH₂)₂)aa -Fb' Formula A-IVa

wherein
- aa is an integer comprised between 1 and 16, in particular between 2 and 8,
- Fb and Fb', identical or different, are representing functions -NH- or -CO-, in particular Fb and Fb' represents -NH-. They would form an amide bond with the aspartate or glutamate unit of the PLG they are linked with.

In a preferred embodiment Q comprises only one Q'.

In a most preferred embodiment Q comprises only one Q' chosen from formula A-III, A-IV and A-IVa.

In one embodiment, the composition according to the invention is characterized in that the co-polyamino acid bearing carboxylate charges and hydrophobic radicals is chosen from the co-polyamino acids of formula A-XXX: 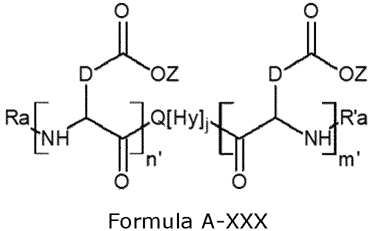 Wherein
- D represents, independently, either a group -CH₂- (aspartic acid) or a group - CH₂-CH₂- (glutamic acid),
- Z represents a cationic entity chosen in the group comprising alkali cations,
- Ra and R'a, identical or different, are either a hydrophobic radical -Hy, or a radical chosen in the group consisting of an H, a C2 to C10 linear acyl group, a C3 to C10 branched acyl group, a benzyl, a terminal "amino acid" unit and a pyroglutamate,
- at least one of Ra and R'a being a hydrophobic radical -Hy, in particular only one of Ra and Ra' is a hydrophobic radical -Hy,
- Q is corresponding to only one Q', in particular chosen from the group consisting of Formula A-III, A-IV or A-IVa
- -Hy is as previously defined,
- n' + m' represents the degree of polymerization DP of the co-polyamino acid, namely the mean number of monomeric units per co-polyamino acid chain and 8 ≤ n' + m' ≤ 50.

According to an embodiment, the backbone "PLG" of the co-polyamino acid bearing carboxylate charges and hydrophobic radicals is as disclosed in WO2019110625 and its US equivalent US2022/040099 which are incorporated by reference.

To be clear, in an embodiment, the co-polyamino acid bearing carboxylate charges and hydrophobic radicals bear the hydrohobic radical as disclosed in the instant specification with the PLG backbone as disclosed in WO2019110625 and its US equivalent US2022/040099.

According to an embodiment, the backbone "PLG" of the co-polyamino acid bearing carboxylate charges and hydrophobic radicals is as disclosed in WO2019/243628 which os incorporated by reference.

To be clear, in an embodiment, the co-polyamino acid bearing carboxylate charges and hydrophobic radicals bear the hydrohobic radical as disclosed in the instant specification with the PLG backbone as disclosed in WO2019/243628.

In one embodiment, the composition according to the invention is characterized in that the co-polyamino acid bearing carboxylate charges and hydrophobic radicals is chosen among the co-polyamino acids of formula XXX, XXXa, XXXa', XXXb, XXXb', XXXb" or A-XXX in which group D is a -CH₂- group (aspartic unit).

In one embodiment, the composition according to the invention is characterized in that the co-polyamino acid bearing carboxylate charges and hydrophobic radicals is chosen from the co-polyamino acids of formula XXX, XXXa, XXXa', XXXb, XXXb', XXXb" or A-XXX in which group D is a -CH₂-CH₂- group (glutamic unit).

In one embodiment, the composition according to the invention is characterized in that R₁ is a radical chosen from the group comprised by a linear acyl group in C₂ to C₁₀, a branched acyl group in C₄ to C₁₀, a benzoyl, a terminal "amino acid" unit and a pyroglutamate.

In one embodiment, the composition according to the invention is characterized in that R₁ is a radical chosen from the group comprised by a linear acyl group in C₂ to C₁₀, or a branched acyl group in C₄ to C₁₀.

In one embodiment, the composition according to the invention is characterized in that n+m is between 10 and 200.

In one embodiment, the composition according to the invention is characterized in that n+m is between 15 and 150.

In one embodiment, the composition according to the invention is characterized in that n+m is between 15 and 100.

In one embodiment, the composition according to the invention is characterized in that n+m is between 15 and 80.

In one embodiment, the composition according to the invention is characterized in that n+m is between 15 and 65.

In one embodiment, the composition according to the invention is characterized in that n+m is between 20 and 60.

In one embodiment, the composition according to the invention is characterized in that n+m is between 20 and 50.

In one embodiment, the composition according to the invention is characterized in that n+m is between 20 and 40.

In a preferred embodiment, the composition comprises a co-polyamino acid according to formula XXXb' or XXXb", in particular XXXb': 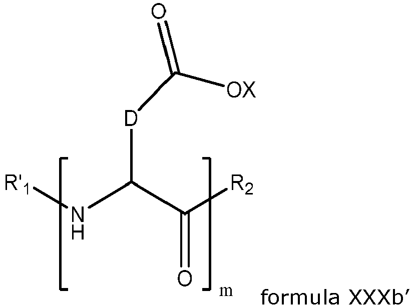 in which m, X, D, R'₁ and R₂ have the definitions given above and R₂ is a hydrophobic radical -Hy,
or 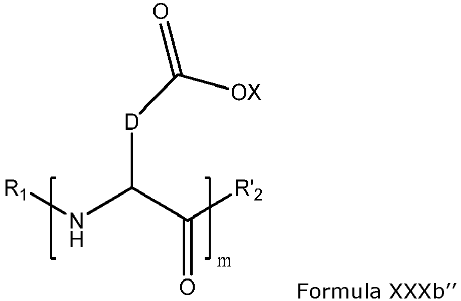 in which m, X, D, R₁ and R'₂ have the definitions given above and R₁ is a hydrophobic radical -Hy, said radical -Hy is chosen from the group of hydrophobic radicals from formula X 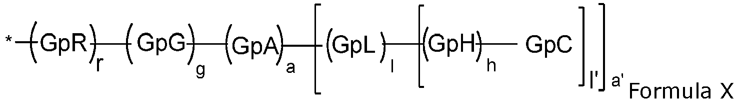 in which:
- r is an integer equal to 0 or to 1, in particular r = 1, and GpR is a radical according to Formula VII 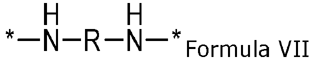
- g = 0,
- a = 0
- a' = 1
- l = 1 and GpL is a radical according to formula XII: 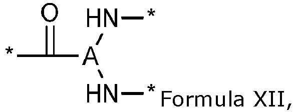
- A is a linear or branched alkyl radical and/or substituted by a radical derived from a saturated, unsaturated or aromatic ring, comprising 1 to 8 carbon atoms, in particular A is a linear or branched alkyl radical comprising 1 to 8 carbon atoms, more particularly A is a linear alkyl radical comprising 5 carbon atoms,
- l' = 2
- h = 1, 2 or 3, in particular h = 1, and GpH is a radical according to Formula XI: 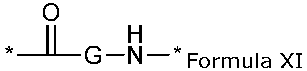
- G is a branched alkyl radical of 1 to 8 carbon atoms, with said alkyl radical bearing one or more free carboxylic acid functions, in particular one free carboxylic acid function,
- GpC is a radical according to formula IX: 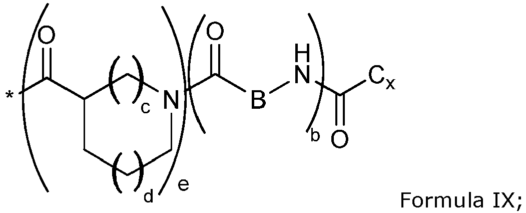 in which b = 0; e = 1; c = 0 or 1; d = 0, 1, or 2; and if c is equal to 0, then d is equal to 1 or to 2; in particular c = 0 and d = 1,
- Cₓ is a linear or branched monovalent alkyl radical, and/or comprising a cyclic part, in which x indicates the number of carbon atoms and when the hydrophobic radical -Hy carries 2 -GpC, then 9 ≤ x ≤ 15, in particular x = 13,
- the hydrophobic radical(s) -Hy according to formula X being bonded to the PLG:
   ∘ via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
   ∘ via a covalent bond between a nitrogen atom of the hydrophobic radical - Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0.01 < M ≤ 0.4, in particular from 0.02 to 0.1,
- when several hydrophobic radicals are borne by a co-polyamino acid, then they are identical or different,
- the degree of polymerization DP in glutamic or aspartic units for PLG chains is comprised from 5 to 250, in particular from 10 to 50,
- free carboxylic acid functions being in the form of an alkali metal salt chosen from the group consisting of Na⁺ and K⁺.

In a most preferred embodiment, the composition comprises a co-polyamino acid as represented below: 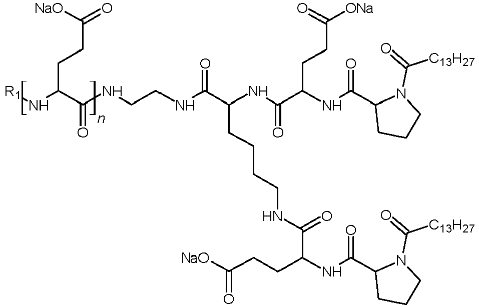 M = 0.02 to 0.1, DP or n = 10 to 50, R₁ = H or pyroglutamate

In a preferred embodiment the composition comprises a co-polyamino acid as defined in the 2 paragraphs above.

According to an embodiment, the co-polyamino acid is as defined in WO2019/110774.

In one embodiment, the composition according to the invention is characterized in that the co-polyamino acid is the result of a polyamino acid obtained by polymerization.

In one embodiment, the composition according to the invention is characterized in that the co-polyamino acid is the result of a polyamino acid obtained by ring-opening polymerization of a N-carboxy anhydride derivative of glutamic acid or of a N-carboxy anhydride derivative of aspartic acid.

In one embodiment, the composition according to the invention is characterized in that the co-polyamino acid is the result of a polyamino acid obtained by ring-opening polymerization of a *N*-carboxyanhydride derivative of glutamic acid or of a *N*-carboxy anhydride derivative of aspartic acid as described in the article from the journal Adv. Polym. Sci. 2006, 202, 1-18 (Deming, T.J.).

In one embodiment, the composition according to the invention is characterized in that the co-polyamino acid is the result of a polyamino acid obtained by ring opening polymerization of a N-carboxy anhydride derivative of glutamic acid.

In one embodiment, the composition according to the invention is characterized in that the co-polyamino acid comes from a polyamino acid obtained by ring-opening polymerization of a N-carboxy anhydride derivative of glutamic acid chosen from the group constituted by *N*-carboxyanhydride methyl glutamate (GluOMe-NCA), N-carboxy anhydride benzyl glutamate (GluOBzl-NCA) and N-carboxy anhydride t-butyl glutamate (GluOtBu-NCA).

In one embodiment, the N-carboxy anhydride glutamic acid derivative is methyl L-glutamate N-carboxy anydride (L-GluOMe-NCA).

In one embodiment, the N-carboxy anhydride glutamic acid derivative is benzyl L-glutamate N-carboxy anydride (L-GluOBzl-NCA).

In one embodiment, the composition according to the invention is characterized in that the co-polyamino acid comes from a polyamino acid obtained by ring opening polymerization of a *N*-carboxyanhydride derivative of glutamic acid or a *N*-carboxyanhydride derivative of aspartic acid using an organo-metallic compound of a transition metal as an initiator as described in the publication Nature 1997, 390, 386-389 (Deming, T. J.).

In one embodiment, the composition according to the invention is characterized in that the co-polyamino acid is the result of a polyamino acid obtained by ring opening polymerization of a *N*-carboxyanhydride derivative of glutamic acid or a *N*-carboxyanhydride derivative of aspartic acid using ammonia or a primary amine as described in French patent FR 2,801,226 (Touraud, F., et al.) and the references cited by this patent.

In one embodiment, the composition according to the invention is characterized in that the co-polyamino acid is the result of a polyamino acid obtained by ring opening polymerization of a *N*-carboxyanhydride derivative of glutamic acid or a *N*-carboxyanhydride derivative of aspartic acid using hexamethyldisilazide as described in the publication J. Am. Chem. Soc. 2007, 129, 14114-14115 (Lu H. ; et al.) or a silylated amine as described in the publication J. Am. Chem. Soc. 2008, 130, 12562-12563 (Lu H. ; et al.).

In one embodiment, the composition according to the invention is characterized in that the process for synthesizing the polyamino acid obtained by ring opening polymerization of a *N*-carboxyanhydride derivative of glutamic acid or a *N-*carboxyanhydride derivative of aspartic acid from which the co polyamino acid results comprises a stage of ester functions hydrolysis.

In one embodiment, this step of ester functions hydrolysis may consist of hydrolysis in an acidic medium or hydrolysis in a basic medium or be carried out by hydrogenolysis.

In one embodiment, this ester groups hydrolysis step is carried out by hydrolysis in an acidic medium.

In one embodiment, this ester groups hydrolysis step is carried out by hydrolysis in a basic medium.

In one embodiment, this ester groups hydrolysis step is carried out by hydrogenolysis.

In one embodiment, the composition according to the invention is characterized in that the co-polyamino acid is the result of a polyamino acid obtained by depolymerization of a polyamino acid of a higher molecular weight.

In one embodiment, the composition according to the invention is characterized in that the co-polyamino acid is the result of a polyamino acid obtained by enzymatic depolymerization of a polyamino acid of a higher molecular weight.

In one embodiment, the composition according to the invention is characterized in that the co-polyamino acid is the result of a polyamino acid obtained by chemical depolymerization of a polyamino acid of a higher molecular weight.

In one embodiment, the composition according to the invention is characterized in that the co-polyamino acid is the result of a polyamino acid obtained by enzymatic and chemical depolymerization of a polyamino acid of a higher molecular weight.

In one embodiment, the composition according to the invention is characterized in that the co-polyamino acid is the result of a polyamino acid obtained by depolymerization of a polyamino acid of a higher molecular weight chosen from the group constituted by sodium polyglutamate and sodium polyaspartate.

In one embodiment, the composition according to the invention is characterized in that the co-polyamino acid is the result of a polyamino acid obtained by depolymerization of a sodium polyglutamate of a higher molecular weight.

In one embodiment, the composition according to the invention is characterized in that the co-polyamino acid is the result of a polyamino acid obtained by depolymerization of a sodium polyaspartate of a higher molecular weight.

In one embodiment, the composition according to the invention is characterized in that the co-polyamino acid is obtained by grafting a hydrophobic group onto a poly-L-glutamate acid or a poly-L-aspartate acid by using the amide bond formation process well-known to the person versed in the art.

In one embodiment, the composition according to the invention is characterized in that the co-polyamino acid is obtained by grafting a hydrophobic group onto a poly-L-glutamate acid or a poly-L-aspartate acid by using the amide bond formation process used for peptide synthesis.

In one embodiment, the composition according to the invention is characterized in that the co-polyamino acid is obtained by grafting a hydrophobic group onto a poly-L-glutamate acid or a poly-L-aspartate acid as described in French patent FR 2,840,614 (Chan, Y.P.; et al.).

In one embodiment, the concentration of co-polyamino acid bearing carboxylate charges and hydrophobic radicals is at most 60 mg/ml.

In one embodiment, the concentration of co-polyamino acid bearing carboxylate charges and hydrophobic radicals is at most 40 mg/ml.

In one embodiment, the concentration of co-polyamino acid bearing carboxylate charges and hydrophobic radicals is at most 20 mg/ml.

In one embodiment, the concentration of co-polyamino acid bearing carboxylate charges and hydrophobic radicals is at most 10 mg/ml.

In one embodiment, the concentration of co-polyamino acid bearing carboxylate charges and hydrophobic radicals is at most 5 mg/ml.

In one embodiment, the concentration of co-polyamino acid bearing carboxylate charges and hydrophobic radicals is at most 2.5 mg/ml.

In one embodiment, the concentration of co-polyamino acid bearing carboxylate charges and hydrophobic radicals is ranging from 2.5 to 60 mg/ml.

In one embodiment, the concentration of co-polyamino acid bearing carboxylate charges and hydrophobic radicals is ranging from 5 to 50 mg/ml.

In one embodiment, the concentration of co-polyamino acid bearing carboxylate charges and hydrophobic radicals is ranging from 7.5 to 40 mg/ml.

In one embodiment, the concentration of co-polyamino acid bearing carboxylate charges and hydrophobic radicals is ranging from 10 to 30 mg/ml.

According to an embodiment, the composition does not comprise a basal insulin for which the isoelectric point, pI, is comprised from 5.8 to 8.5.

According to an embodiment, the composition does not comprise insulin glargine.

According to an embodiment, the composition does not comprise human amylin.

According to an embodiment, the composition does not comprise pramlintide.

According to an embodiment, the composition does not comprise glucagon.

According to an embodiment, the composition does not comprise human glucagon.

Semaglutide is a GLP-1 receptor agonist also known as N<626>-{18-[N-(17-carboxyheptadecanoyl)-L-y-glutamyl]-10-oxo-3,6,12,15-tetraoxa-9,18-diazaoctadecanoyl}-[8-(2-amino-2-propanoic acid),34-L-arginine]human glucagon-like peptide 1 (7-37). Semaglutide was described in WO2006/097537 and in J. Med. Chem. 2015, 58, 18, 7370-7380 and may be manufactured using methods well known to the person skilled in the art, such as that briefly described in WO2006/097537, Example 4.

Semaglutide may be present in the composition in its fully or partly ionized form; for example, one or more carboxylic acid groups (-COOH) may be deprotonated into the carboxylate group -COO⁻ and/or one or more amino groups (-NH₂) may be protonated into the -NH₃⁺ group.

Semaglutide may be in the form of a salt, preferably a pharmaceutically acceptable salt.

When the potency of semaglutide was tested according to the procedure described in WO/2022/129526, Assay 1, semaglutide had an EC50 value of about 5.5 pM (see WO/2022/129526, Tables 4b and 4c). The concentration of semaglutide in the pharmaceutical formulation disclosed herein may be from about 0.25 mg/ml to about 22 mg/ml.

The concentration of semaglutide in the compositions disclosed herein may be from about 0.25 mg/ml to about 22 mg/ml.

In an embodiment, the semaglutide is comprised in a concentration ranging from 0.3 to 20 mg/ml.

In an embodiment, the semaglutide is comprised in a concentration ranging from 0.25 to 0.5 mg/ml, in particular is about 0.37 mg/ml.

In an embodiment, the semaglutide is comprised in a concentration ranging from 0.5 to 1.0 mg/ml, in particular is about 0.75 mg/ml.

In an embodiment, the semaglutide is comprised in a concentration ranging from 1.0 to 1.5 mg/ml, in particular is about 1.25 mg/ml.

In an embodiment, the semaglutide is comprised in a concentration ranging from 1.5 to 2.0 mg/ml.

In an embodiment, the semaglutide is comprised in a concentration ranging from 2.0 to 2.5 mg/ml.

In an embodiment, the semaglutide is comprised in a concentration ranging from 2.5 to 3.0 mg/ml.

In an embodiment, the semaglutide is comprised in a concentration ranging from 3.0 to 3.5 mg/ml, in particular is about 3.2 mg/ml.

In an embodiment, the semaglutide is comprised in a concentration ranging from 3.5 to 4.0 mg/ml.

In an embodiment, the semaglutide is comprised in a concentration ranging from 4.0 to 5.0 mg/ml.

In an embodiment, the semaglutide is comprised in a concentration ranging from 5.0 to 7.5 mg/ml.

In an embodiment, the semaglutide is comprised in a concentration ranging from 7.5 to 10 mg/ml.

In an embodiment, the semaglutide is comprised in a concentration ranging from 10 to 15 mg/ml.

In an embodiment, the semaglutide is comprised in a concentration ranging from 15 to 20 mg/ml.

In an embodiment, the semaglutide is comprised in a concentration ranging from 20 to 25 mg/ml.

Semaglutide has a theoretically calculated isoelectric point of about 4.37.

In a preferred embodiment, the cagrilintide define cagrilintide or a biologically active metabolite or degradation product of cagrilintide.

In a most preferred embodiment, cagrilintide means cagrilintide alone, i.e not comprising its biologically active metabolites or degradation products.

Cagrilintide has a theoretically calculated isoelectric point of about 8.56.

Cagrilintide, also called AM833, is disclosed as Example 53 in WO2012/168432: N-alpha-[(S)-4-Carboxy-4-(19-carboxynonadecanoylamino)butyryl]-[Glu14,Arg17,Pro37]-pramlintide. Cagrilintide may be prepared as described in WO2012/168432, pages 153-155.

Cagrilintide may be in the form of a salt, preferably a pharmaceutically acceptable salt.

A biologically active metabolite or degradation product of cagrilintide may have an aspartate (Asp) in position 21 or 22. A biologically active metabolite or degradation product of cagrilintide may have an iso-aspartate (iso-Asp) in position 21 or 22.

When the potency of cagrilintide was tested using the procedure described in WO2022129526, Assay 2, cagrilintide had an EC50 value of about 11 pM (WO2022/129526, Tables 4b and 4c).

In an embodiment, the concentration of cagrilintide is going from about 0.25 mg/ml to about 22 mg/ml.

In an embodiment, the concentration of cagrilintide is going from 0.3 to 20 mg/ml.

In an embodiment, the concentration of cagrilintide is going from about 0.25 to 0.5 mg/ml, in particular is about 0.37 mg/ml.

In an embodiment, the concentration of cagrilintide is going from about 0.5 to 1.0 mg/ml, in particular is about 0.75 mg/ml.

In an embodiment, the concentration of cagrilintide is going from about 1.0 to 1.5 mg/ml, in particular is about 1.25 mg/ml.

In an embodiment, the cagrilintide is comprised in a concentration ranging from 1.5 to 2.0 mg/ml.

In an embodiment, the cagrilintide is comprised in a concentration ranging from 2.0 to 2.5 mg/ml.

In an embodiment, the cagrilintide is comprised in a concentration ranging from 2.5 to 3.0 mg/ml.

In an embodiment, the cagrilintide is comprised in a concentration ranging from 3.0 to 3.5 mg/ml, in particular about 3.25 mg/ml.

In an embodiment, the cagrilintide is comprised in a concentration ranging from 3.5 to 4.0 mg/ml.

In an embodiment, the cagrilintide is comprised in a concentration ranging from 4.0 to 5.0 mg/ml.

In an embodiment, the cagrilintide is comprised in a concentration ranging from 5.0 to 7.5 mg/ml.

In an embodiment, the cagrilintide is comprised in a concentration ranging from 7.5 to 10 mg/ml.

In an embodiment, the cagrilintide is comprised in a concentration ranging from 10 to 15 mg/ml.

In an embodiment, the cagrilintide is comprised in a concentration ranging from 15 to 20 mg/ml.

In an embodiment, the cagrilintide comprised in a concentration ranging from 20 to 25 mg/ml.

In an embodiment, the weight ratio semaglutide / cagrilintide- is going from 0.1 to 10.

In an embodiment, the weight ratio semaglutide / cagrilintide is going from 0.2 to 5.

In an embodiment, the weight ratio semaglutide / cagrilintide is going from 0.3 to 3.

In an embodiment, the weight ratio semaglutide / cagrilintide is going from 0.5 to 2.

In an embodiment, the weight ratio semaglutide / cagrilintide is going from 0.8 to 1.2.

In an embodiment, the weight ratio semaglutide / cagrilintide is going from 0.9 to 1.1.

In an embodiment, the weight ratio semaglutide / cagrilintide is about 1.

In an embodiment the compositions comprise 3.2 mg of cagrilintide and 3.2 mg of semaglutide.

The pH of compositions according to the invention is comprised in a range from 5.0 to 6.0.

According to an embodiment the pH of 6.0 can be disclaimed from the ranges of pH.

In an embodiment the pH is comprised in a range from 5.0 to 5.9.

In an embodiment the pH is comprised in a range from 5.1 to 5.9.

In an embodiment the pH is comprised in a range from 5.2 to 5.9.

In an embodiment the pH is comprised in a range from 5.2 to 5.8.

In an embodiment the pH is comprised in a range from 5.2 and 5.7.

In an embodiment the pH is comprised in a range from 5.2 and 5.6.

In an embodiment the pH is comprised in a range from 5.3 and 5.5.

In an embodiment the pH is about 5.4.

In one embodiment, the hydrophobic radical to semaglutide and cagrilintide molar ratio [Hy]/ [semaglutide] + [cagrilintide] ≤ 6.

In one embodiment, the hydrophobic radical to semaglutide and cagrilintide molar ratio [Hy]/ [semaglutide] + [cagrilintide] ≤ 5.

In one embodiment, the hydrophobic radical to semaglutide and cagrilintide molar ratio [Hy]/ [semaglutide] + [cagrilintide] ≤ 4.

In one embodiment, the hydrophobic radical to semaglutide and cagrilintide molar ratio [Hy]/ [semaglutide] + [cagrilintide] ≤ 3.

In one embodiment, the hydrophobic radical to semaglutide and cagrilintide molar ratio [Hy]/ [semaglutide] + [cagrilintide] ≥ 1.

In one embodiment, the hydrophobic radical to semaglutide and cagrilintide molar ratio [Hy]/ [semaglutide] + [cagrilintide] ≥ 1.5.

In one embodiment, the hydrophobic radical to semaglutide and cagrilintide molar ratio [semaglutide] + [cagrilintide] ≥ 2.

In one embodiment, the hydrophobic radical to semaglutide and cagrilintide molar ratio [Hy]/ [semaglutide] + [cagrilintide] ≥ 2.5.

In one embodiment, the compositions according to the invention also comprise buffers.

In one embodiment, the composition according to the invention comprises buffers at concentrations between 0 and 100 mM.

In one embodiment, the composition according to the invention comprises buffers at concentrations between 15 and 50 mM.

In one embodiment, compositions according to the invention comprise a buffer chosen from the group constituted by a phosphate buffer, Tris (trishydroxymethyl-aminomethane).

In one embodiment, the buffer is sodium phosphate.

In one embodiment, the buffer is Tris (trishydroxymethyl-aminomethane).

In one embodiment, the buffer is sodium citrate.

In one embodiment, the compositions according to the invention also comprise a preservative.

In one embodiment, the preservatives are chosen from the group constituted by m-cresol and phenol, alone or in mixture.

In one embodiment, the concentration of the preservatives is between 10 and 50 mM.

In one embodiment, the concentration of the preservatives is between 10 and 40 mM.

In one embodiment, the compositions according to the invention also comprise a surfactant.

In one embodiment, the surfactant is chosen from the group constituted by propylene glycol and polysorbate.

Compositions according to the invention may also comprise additives such as osmotic agents.

In one embodiment, the osmotic agents are chosen from the group constituted by glycerin, sodium chloride, mannitol and glycine.

Compositions according to the invention may also comprise any pharmacopoeia compatible excipients which are also compatible with cagrilintide and semaglutide, at the usage concentrations.

The compositions or formulations disclosed herein are suitable for parenteral injection, in particular for subcutaneous injection.

In the case of local and systemic releases, the administration pathways foreseen are intravenous, subcutaneous, intra-dermal or intramuscular.

In a preferred embodiment the administration is subcutaneous.

In one embodiment, the composition according to the invention is characterized in that it is administered twice a week.

In one embodiment, the composition according to the invention is characterized in that it is administered once a week.

In one embodiment, the composition according to the invention is characterized in that it is administered twice a month.

The invention also relates to single-dose formulations at a pH between 5.2 and 6.0 and comprising cagrilintide, semaglutide and co-polyamino acid as defined above.

In one embodiment, the formulations are in the form of an injectable solution.

If necessary, the composition of the mixture of cagrilintide, semaglutide and the co-polyamino acid is adjusted with excipients such as glycerin, m-cresol, and polysorbate (Tween^{®}) by addition of concentrated solutions of these excipients to the mixture. If necessary, the pH of the preparation is adjusted to a pH between 5.0 and 6.0.

According to an embodiment, the composition is in the form of an aqueous injectable solution, whose pH is comprised from 5.0 to 6.0, in particular 5.1 to 5.9, comprising:
- semaglutide,
- cagrilintide, and
- a co-polyamino acid (PLG) bearing carboxylate charges and hydrophobic radicals Hy having the following Formula 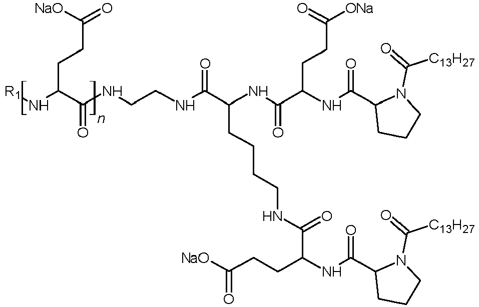
M = 0.02 to 0.1, DP = 10 to 50, R₁ = H or pyroglutamate.

According to an embodiment, the composition is in the form of an aqueous injectable solution, whose pH is comprised from 5.2 to 5.8, comprising:
- semaglutide,
- cagrilintide, and
- a co-polyamino acid (PLG) bearing carboxylate charges and hydrophobic radicals Hy having the following Formula: 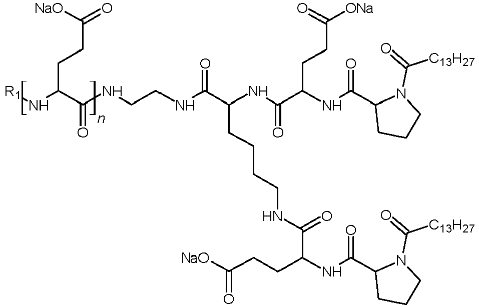
M = 0.02 to 0.1, DP or n = 10 to 50, R₁ = H or pyroglutamate.

The instant invention further concerns compositions for use as a medicament.

In an embodiment, the invention concerns a composition, in the form of an aqueous injectable solution, for use as a medicament, whose pH is comprised from 5.0 to 6.0, in particular from 5.1 to 5.9, comprising:
- semaglutide,
- cagrilintide, and
- a co-polyamino acid (PLG) bearing carboxylate charges and hydrophobic radicals Hy having the following Formula: 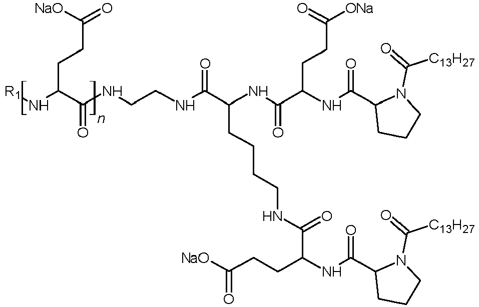
M = 0.02 to 0.1, n = 10 to 50, R₁ = H or pyroglutamate.

In an embodiment, the invention concerns a composition, in the form of an aqueous injectable solution, for use as a medicament, whose pH is comprised from 5.2 to 5.8, comprising:
- semaglutide,
- cagrilintide, and
- a co-polyamino acid (PLG) bearing carboxylate charges and hydrophobic radicals Hy having the following Formula: 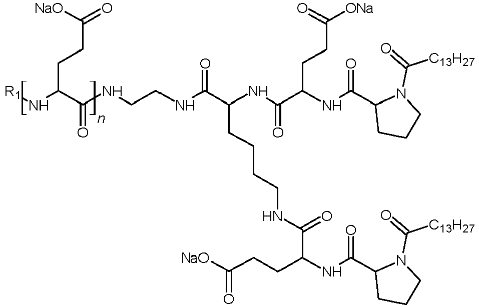
M = 0.02 to 0.1, n = 10 to 50, R₁ = H or pyroglutamate.

The invention also concerns compositions for use in a method of treatment of overweight or obesity, diabetes, cardio-vascular disease(s), non-alcoholic steatohepatitis (NASH), and/or cognitive impairment, such as caused by Alzheimer Disease (AD). Diabetes, overweight and obesity could be with or without one or more associated comorbidities.

In an embodiment, compositions are for use in a method of treatment of overweight or obesity.

In an embodiment, compositions are for use in a method of treatment of diabetes.

In an embodiment, compositions are for use in a method of treatment of Diabetes Type 1.

In an embodiment, compositions are for use in a method of treatment of Diabetes Type 2.

In an embodiment, compositions are for use in a method of treatment of overweight or obesity and diabetes.

In an embodiment, compositions are for use in a method of treatment of overweight or obesity and Diabetes Type 1.

In an embodiment, compositions are for use in a method of treatment of overweight or obesity and Diabetes Type 2.

In an embodiment, compositions are for use in a method of treatment of cardio-vascular disease(s).

In an embodiment, compositions are for use in a method of treatment of non-alcoholic steatohepatitis (NASH).

The invention further concerns a method for the preparation of a stable, injectable aqueous solutions of semaglutide comprising the step of mixing a co-polyamino acid as previously defined with semaglutide.

In an embodiment, the method is for the preparation of a stable, injectable aqueous solutions of semaglutide comprising the step of mixing a co-polyamino acid (PLG) bearing carboxylate charges and hydrophobic radicals Hy having the following Formula: 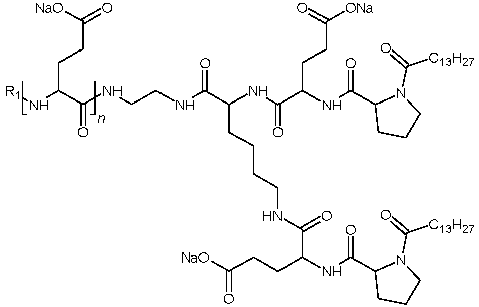
M = 0.02 to 0.1, n = 10 to 50, R₁ = H or pyroglutamate,
with semaglutide.

### EXAMPLES

### Part A - Chemistry

Precursors of Hydrophobic Grafts and Co-polyamino acids bearing Carboxylate Charges and Hydrophobic Grafts could be obtained as described in US2020306379A1 and in WO2019110773.

| **No.** | **CO-POLYAMINO ACIDS BEARING CARBOXYLATE CHARGES AND HYDROPHOBIC RADICALS** |
|---|---|
| A1 | 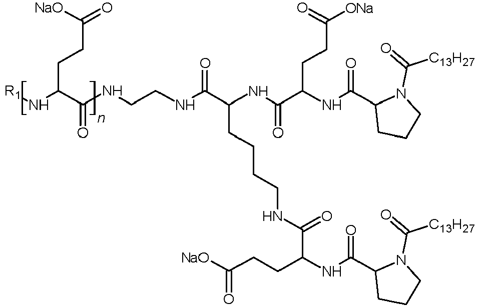 |
| | M = 0.05, n = 20 |
| | R₁ = H or pyroglutamate |

In particular Co-polyamino acid A1 corresponds to Co-polyamino acid B22 from US2020306379A1 and WO2019110773.

### Part B - Compositions

### Example B1: Stock Solutions

### Example B1a: Solutions of Cagrilintide

Solutions of cagrilintide have been prepared by weighing the adequate amount of cagrilintide powder and adding the desired amount of water to reach the desired concentration. A clear solution was obtained by gentle mixing via vial inversions.

### Example B1b: Solutions of Semaglutide

Solutions of semaglutide have been prepared by weighing the adequate amount of semaglutide powder and adding the desired amount of water to reach the desired concentration. A clear solution was obtained by gentle mixing via vial inversions.

### Example B1b': Solutions of Tirzepatide

Solutions of tirzepatide have been prepared by weighing the adequate amount of tirzepatide powder and adding the desired amount of water to reach the desired concentration. A clear solution was obtained by gentle mixing via vial inversions.

### Example B1c: Solutions of Co-polyamino acid A1

Solutions of co-polyamino acid A1 were prepared by weighing the adequate amount of co-polyaminoacid A1 powder, then dissolving it in water. An adjustement to pH 5.4 using NaOH and HCl 1M was done prior to adding water to reach the desired concentration. A clear solution was obtained.

### Example B2: Compositions of the invention comprising Cagrilintide and Semaglutide or Cagrilintide and Tirzepatide

### Example B2a: Compositions of Cagrilintide, Semaglutide and Co-polvamino acid A1

Solutions according to the invention were prepared as follows:
- Addition of concentrated aqueous solutions of excipients (i.e. m-cresol (m-cr), mannitol (man), phenol) to the solution of co-polyaminoacid A1 (PLG-A1).
- pH adjustment of this solution to the desired pH when needed (5.4;5.8)
- addition of semaglutide (Sema) solution
- addition of cagrilintide (Cagri) solution
- final pH adjustment if needed, using NaOH 1N or HCl 1N
- addition of water to reach the final desired volume and concentrations.

A final filtration onto 0.22µm filter was done prior to filling.

The following compositions have been prepared:

| Example | [Sema] (mg/ml) | [Cagri] (mg/ml) | PLG-A1 (mg/ml) | Excipients | pH |
|---|---|---|---|---|---|
| B2a-2 | 3.2 | 3.2 | 15 | man 36 mg/mL | 5.4;5.8 |
| B2a-3 | 3.2 | 3.2 | 15 | man 36 mg/mL, m-cr 35 mmol/L | 5.4;5.8 |
| B2a-4 | 3.2 | 3.2 | 15 | man 36 mg/mL, m-cresol 35 mmol/L | 5.6 |
| B2a-5 | 3.2 | 3.2 | 10 | man 29 mg/mL, phenol 70 mmol/L | 5.8 |

Using these compositions, 3 mL cartridges have been filled with 0.5 mL of formulation.

### Example B2b: Compositions of Cagrilintide, Tirzepatide and Co-polyamino acid A1

Solutions according to the invention were prepared as follows:
- Addition of concentrated aqueous solutions of excipients (i.e. phenol, mannitol (man)) to the solution of co-polyaminoacid A1 (PLG-A1).
- pH adjustment of this solution to the desired pH when needed (5.4;5.8)
- addition of tirzepatide (Tirze) solution
- addition of cagrilintide (Cagri) solution
- final pH adjustment if needed, using NaOH 1N or HCl 1N
- addition of water to reach the final desired volume and concentrations.

A final filtration onto 0.22µm filter was done prior to filling.

The following compositions have been prepared:

| Example | [Tirze] (mg/ml) | [Cagri] (mg/ml) | PLG-A1 (mg/ml) | Excipients | pH |
|---|---|---|---|---|---|
| B2b-1 | 6.7 | 3.2 | 12.5 | man 32 mg/mL, phenol 70mmol/L | 5.8 |
| B2b-2 | 13.3 | 3.2 | 20 | man 27 mg/mL, phenol 70 mmol/L | 5.8 |

### Part C - Stability

### Example C1: Monitoring stability

### Monitoring of Physical Stability

The cartridges containing the compositions were placed at 30°C. A physical stress was applied to the cartridges in the form of 20 daily container manual inversions (40 inversions on Mondays ans Fridays, none on Saturdays ans Sundays).

At times specified in the following tables, one cartridge per composition was retrieved for physical stability assessment. Physical stability was monitored by visual inspection under USP<790> conditions, as well as by MFI sub-visible particle content measurement.

For MFI measurements, the cartridges were opened, and 100µL were sampled without moving the plunger. These 100µL were added to 900µL of water filtered onto 0.22µm filter to yield 1mL of sample for MFI measurement. Sub-visible particle content per mL was then measured on the diluted sample. Using the dilution factor of 10, the number of sub-visible particles per mL of formulation was then calculated.

### Monitoring of Chemical Stability

For the monitoring of chemical stability, cartridges were placed at 37°C. At times specified in the following tables, one cartridge per composition was retrieved for analysis.

Chemical stability was assessed both by RP-HPLC or RP-UPLC analysis to monitor each peptide's purity, and by SEC-HPLC to monitor the formation of high molecular weight products (HMWP)

### Example C2: Stability Results

### Example C2-1: Stability study on Cagrilintide / Semaglutide compositions

Results of visual inspection and MFI measurements done on day 14 and 26 of accelerated physical stability at 30°C studies are presented below:

| Formulation - pH | Part >5µm (/mL) | | Part>10µm (/mL) | | Part>25µm (/mL) | | Visual inspection | |
|---|---|---|---|---|---|---|---|---|
| | T14D | T26D | T14D | T26D | T14D | T26D | T14D | T26D |
| B2a-2 - 5.4 | 161 | 161 | 46 | 69 | 0 | 46 | clear | clear |
| B2a-2 - 5.8 | 2799 | 1399 | 1055 | 413 | 321 | 183 | clear | clear |
| B2a-3 - 5.4 | 160 | 1054 | 0 | 206 | 0 | 46 | clear | clear |
| B2a-3 - 5.8 | 321 | 390 | 46 | 161 | 0 | 92 | clear | clear |

These formulations appeared stable during this accelerated stability study, with visual aspect and sub-visible particle amounts remaining satisfactory.

Results of the chemical stability (RP-HPLC and SEC-HPLC methods) are presented below.

| Formulation - pH | Cagrilintide impurity levels (%, delta vs T0) | | Semaglutide impurity levels (%, delta vs T0) | | HMWP content (%, delta vs T0) | |
|---|---|---|---|---|---|---|
| | T14D | T26D | T14D | T26D | T14D | T26D |
| B2a-2 - 5.4 | +1.8 | +2.2 | +1.3 | +2.6 | +0.0 | +0.1 |
| B2a-2 - 5.8 | +1.8 | +2.4 | +1.2 | +2.3 | +0.0 | +0.1 |
| B2a-3 - 5.4 | +0.8 | +1.4 | +1.4 | +2.6 | +0.1 | +0.2 |
| B2a-3 - 5.8 | +1.5 | +1.9 | +1.3 | +2.3 | +0.1 | +0.3 |

As can be seen from the table above, the level of impurities after 26 days at 37°C is below 2.5% for cagrilintide and below 3% for semaglutide.

For comparison, Novo's application WO2023/183067 disclosed a higher level of impurity increase at 37°C at 28 days for cagrilintide and for semaglutide.

### Example C2-2: Stability study on Cagrilintide / Semaglutide compositions

Results of visual inspection done on day 14, 28 and 42 of accelerated stability at 37°C studies are presented below:

| Visual aspect at | T0 | T14D | T28D | T42D |
|---|---|---|---|---|
| B2a-4 | Clear, colorless | Clear, colorless | Clear, colorless | Clear, colorless |
| B2a-5 | Clear, colorless | Clear, colorless | Clear, colorless | Clear, colorless |

These formulations appeared stable during this accelerated stability study, with visual aspect remaining satisfactory during the storage period.

Results of the chemical stability (RP-UPLC and SEC-HPLC methods) are presented below.

| Formulation | Cagrilintide impurity levels (%, delta vs T0) | | | Semaglutide impurity levels (%, delta vs T0) | | | HMWP content (%, delta vs T0) | | |
|---|---|---|---|---|---|---|---|---|---|
| | T14D | T28D | T42D | T14D | T28D | T42D | T14D | T28D | T42D |
| B2a-4 | +2.9 | +6.8 | +9.1 | +2.9 | +5.7 | +7.9 | +0.2 | +0.4 | +0.6 |
| B2a-5 | +2.8 | +6.5 | +9.8 | +2.8 | +5.6 | +7.6 | +0.2 | +0.6 | +0.9 |

As can be seen from the table above, the increase of impurities after 42 days at 37°C is around 10% for cagrilintide and 8% for semaglutide. The increase of HMWP content remains < 1% after 42 days at 37°C.

For comparison, Novo's application WO2023/183067 disclosed a higher level of impurity increase at 37°C for cagrilintide.

### Example C2-3: Stability study on Cagrilintide / Tirzepatide compositions

Results of visual inspection done on day 14,28 and 45 of accelerated physical stability at 30°C studies are presented below:

| Visual aspect at | T0 | T14D | T28D | T45D |
|---|---|---|---|---|
| B2b-1 | Clear, colorless | Clear, colorless | Clear, colorless | Clear, colorless |
| B2b-1 | Clear, colorless | Clear, colorless | Clear, colorless | Clear, colorless |

These formulations appeared stable during this accelerated stability study, with visual aspect remaining satisfactory during the storage period.

Results of the chemical stability (RP-UPLC and SEC-HPLC methods) are presented below.

| Formulation | Cagrilintide impurity levels (%, delta vs T0) | | | Tirzepatide impurity levels (%, delta vs T0) | | | HMWP content (%, delta vs T0) | | |
|---|---|---|---|---|---|---|---|---|---|
| | T14D | T28D | T45D | T14D | T28D | T45D | T14D | T28D | T45D |
| B2b-1 | +4.3 | +8.2 | +11.8 | +1.3 | +2.8 | +3.8 | +0.1 | +0.3 | +0.5 |
| B2b-2 | +4.4 | +9.1 | +13.7 | +1.6 | +3.0 | +4.0 | +0.0 | +0.2 | +0.3 |

As can be seen from the table above, the increase of impurities after 45 days at 37°C was less than 14% for cagrilintide and less or equal to 4% for tirzepatide. The increase of HMWP content remained less 1% after 45 days at 37°C.

### Part D - Pharmacokinetics (PK)

### Example D1: Pharmacokinetic Study of composition B2a-5 compared to separate injections of cagrilinide and semaglutide

A Pharmacokinetic (PK) study was conducted in Göttingen mini pigs to evaluate the pharmacokinetics (PK) of cagrilintide and semaglutide after subcutaneous (sc) administration of a single injection of the composition described in example B2a-5 or of separate simultaneous injections of cagrilintide and semaglutide.

Two parallel groups of 6 female mini pigs, weighing approximately 50 kg, were injected with about 150 µL of the composition B2a-5 or 150 µL of semaglutide formulation and 150 µL of cagrilintide formulation. Concentrations of APIs were the same in the composition B2a-5 and formulations of cagrilintide and semaglutide. Blood was sampled at predefined time points up to 432h.

Plasma concentrations of cagrilintide and semaglutide were determined using an LC-MS/MS method. Non-compartimental PK analysis, using individual plasma concentration-time profiles, was performed.

Composition B2a-5 showed an exposure similar to separate injections for cagrilintide and for semaglutide. Ratio of mean AUCinf/dose were of 0.93 for cagrilintide and 1.06 for semaglutide. Moreover, no difference was observed in terminal half-life. Ratio were 0.97 for cagrilintide and 0.99 for semaglutide.

In addition, the absorption rate of the APIs was slowed down when administered as composition B2a-5. Indeed, delayed of median Tmax were observed for cagrilintide and semaglutide (from 6h to 36h and from 8h to 36h respectively). A large decrease of Cmax was also observed for cagrilintide. The ratio of mean Cmax/dose was 0.64.

These results show that composition B2a-5 allows single injection of cagrilintide and semaglutide. Moreover, the slowing down of the absorption rate of APIs and/or decreasing of Cmax could lead to interesting advantages (e.g. reducing side effects).

## Claims

1. A composition, in the form of an aqueous injectable solution, whose pH is comprised from 5.0 to 6.0, comprising:
- semaglutide
- cagrilintide, and
- a co-polyamino acid (PLG) bearing carboxylate charges and hydrophobic radicals Hy, said co-polyamino acid consisting of glutamic or aspartic units, and said hydrophobic radicals Hy being according to formula X below: 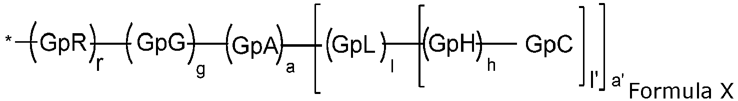 in which
- GpR is chosen from the radicals according to formulas VII, VII' or VII': 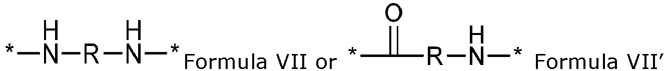 or 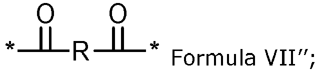
- identical or different GpG and GpH are chosen from the radicals according to formulas XI or XI': 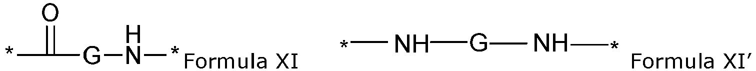
- GpA is chosen from the radicals according to formula VIII: 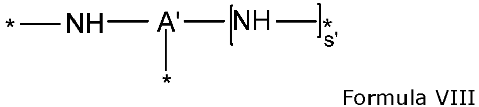 in which A' is chosen from the radicals according to formula VIII', VIII" or VIII‴: 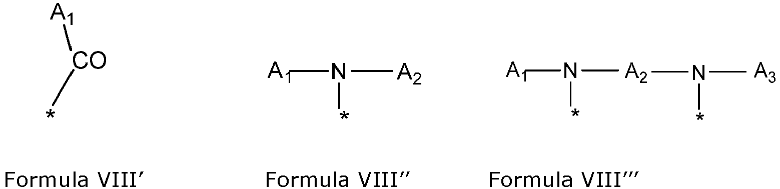
- GpL is chosen from the radicals according to formula XII: 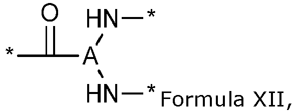
- GpC is a radical according to formula IX: 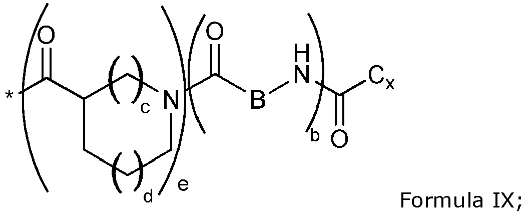 the * indicates the attachment sites of the different groups bonded by the amide functions;
- a is an integer equal to 0 or to 1 and a' = 1 if a = 0 and a' = 1, 2 or 3 if a = 1;
- a' is an integer equal to 1, to 2, or to 3
- b is an integer equal to 0 or to 1;
- c is an integer equal to 0 or to 1, and if c is equal to 0, then d is equal to 1 or to 2;
- d is an integer equal to 0, to 1, or to 2;
- e is an integer equal to 0 or to 1;
- g is an integer equal to 0, to 1, to 2, to 3, to 4, to 5 or to 6;
- h is an integer equal to 0, to 1, to 2, to 3, to 4, to 5 or to 6;
- I is an integer equal to 0 or 1 and I' = 1 if I = 0, and I' = 2 if I = 1;
- r is an integer equal to 0, to 1, or to 2, and
- s' is an integer equal to 0 or 1, and
- if e is different from 0, then at least one of g, h or I is different from 0;
- A, A₁, A₂ and A₃, identical or different, are linear or branched alkyl radicals, and/or substituted by a radical derived from a saturated, unsaturated or aromatic ring, comprising 1 to 8 carbon atoms.
- B is a linear or branched alkyl radical and/or comprising an aromatic nucleus, comprising 1 to 9 carbon atoms or an unsubstituted ether or polyether radical comprising from 4 to 14 carbon atoms and from 1 to 5 oxygen atoms;
- Cₓ is a linear or branched monovalent alkyl radical, and/or comprising a cyclic part, in which x indicates the number of carbon atoms and:
▪ when the hydrophobic radical -Hy carries 1 -GpC, then 9 ≤ x ≤ 25,
▪ when the hydrophobic radical -Hy carries 2 -GpC, then 9 ≤ x ≤ 15,
▪ when the hydrophobic radical -Hy carries 3 -GpC, then 7 ≤ x ≤ 13,
▪ when the hydrophobic radical -Hy carries 4 -GpC, then 7 ≤ x ≤ 11,
▪ when the hydrophobic radical -Hy carries at least 5 -GpC, then 6 ≤ x ≤ 11,
- G is a branched alkyl radical of 1 to 8 carbon atoms, with said alkyl radical bearing one or more free carboxylic acid functions,
- R is a radical chosen from the group consisting of a divalent alkyl radical, linear or branched, comprising 1 to 12 carbon atoms, a divalent alkyl radical, linear or branched, comprising 1 to 12 carbon atoms bearing one or more -CONH₂ functions, or a unsubstituted ether or polyether radical comprising from 4 to 14 carbon atoms and 1 to 5 oxygen atoms:
- the hydrophobic radical(s) -Hy according to formula X being bonded to the PLG:
- via a covalent bond between a carbonyl of the hydrophobic radical -Hy and a nitrogen atom borne by the PLG, thus forming an amide function derived from the reaction of an amine function borne by the PLG and an acid function borne by the precursor -Hy' of the hydrophobic radica -Hy, or
- via a covalent bond between a nitrogen atom of the hydrophobic radical -Hy and a carbonyl borne by the PLG, thus forming an amide function derived from the reaction of an amine function of the precursor -Hy' of the hydrophobic -Hy radical and an acid function borne by the PLG,
- the ratio M between the number of hydrophobic radicals and the number of glutamic or aspartic units being from 0 < M ≤ 0.5;
- when several hydrophobic radicals are borne by a co-polyamino acid, then they are identical or different,
- the degree of polymerization DP in glutamic or aspartic units for PLG chains is comprised from 5 to 250;
- free carboxylic acid functions being in the form of an alkali metal salt chosen from the group consisting of Na⁺ and K⁺.

2. A composition according to Claim 1, **characterized in that** the semaglutide is at a concentration of about 0.25 mg/ml to about 22 mg/ml.

3. A composition according Claim 1 or 2, **characterized in that** it comprises cagrilintide at a concentration of about 0.25 mg/ml to about 22 mg/ml.

4. A composition according any one of Claims 1 to 3, **characterized in that** the co-polyamino acid bearing carboxylate charges and hydrophobic radicals is chosen from the co-polyamino acids according to formula XXXa' below: 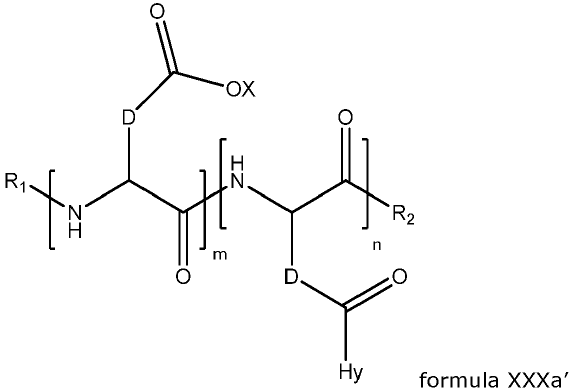 in which
- D represents, independently, either a -CH₂- group (aspartic unit) or a -CH₂-CH₂-group (glutamic unit),
- Hy is a hydrophobic radical chosen from the hydrophobic radicals according to formula X, in which r=1 and GpR is a radical according to formula VII,
- R₁ is a hydrophobic radical chosen from the hydrophobic radicals according to formula X in which r=0 or r=1 and GpR is a radical according to formula VII', or a radical chosen from the group consisting of an H, a linear acyl group in C2 to C10, a branched acyl group in C4 to C10, a benzoyl, a terminal "amino acid" unit and a pyroglutamate,
- R₂ is a hydrophobic radical chosen from the hydrophobic radicals according to formula X in which r=1 and GpR is a radical according to formula VII, or a -NR'R" radical, R' and R", either identical or different, being chosen from the group comprised by H, linear or branched or cyclic alkyls in C2 to C10, or benzyl, and said R' and R" alkyls which may form together one or more carbonated, saturated, unsaturated and/or aromatic cycle(s), and/or may comprise hetero-atoms, chosen from the group comprised of O, N and S;
- X represents a cationic entity chosen from the group comprising alkaline cations;
- n + m represents the degree of polymerization DP of the co-polyamino acid, namely, the average number of monomeric units per co-polyamino acid chain and 5 ≤ n + m ≤ 250.

5. A composition according any one of Claims 1 to 4, **characterized in that** the co-polyamino acid bearing carboxylate charges and hydrophobic radicals is chosen from the co-polyamino acids according to formula XXXa' in which n = 0 according to formula XXXb below: 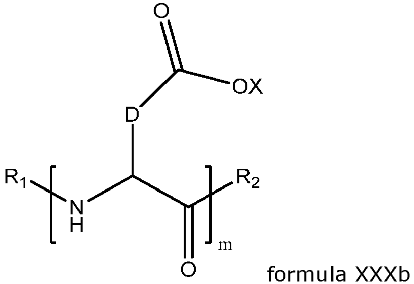 in which m, X, D, R₁ and R₂ have the definitions given in Claim 8 and at least R₁ or R₂ is hydrophobic radical according to formula X.

6. The composition according to according any one of Claims 1 to 5, **characterized in that** the co-polyamino acid bearing carboxylate charges and hydrophobic radicals is chosen from the co-polyamino acids according to formula XXXb in which R₂ is a hydrophobic radical according to formula X in which r = 1 and GpR is according to formula VII.

7. A composition according any one of Claims 1 to 6, **characterized in that** the co-polyamino acid bearing carboxylate charges and hydrophobic radicals is chosen from the co-polyamino acids according to formula co-polyamino acids of formula A-XXX: 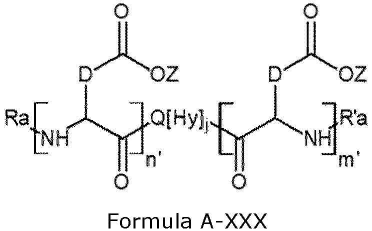 Wherein
- D represents, independently, either a group -CH₂- (aspartic acid) or a group - CH₂-CH₂- (glutamic acid),
- Z represents a cationic entity chosen in the group comprising alkali cations,
- Ra and R'a, identical or different, are either a hydrophobic radical -Hy, or a radical chosen in the group consisting of an H, a C2 to C10 linear acyl group, a C3 to C10 branched acyl group, a benzyl, a terminal "amino acid" unit and a pyroglutamate,
- at least one of Ra and R'a being a hydrophobic radical -Hy, in particular both Ra and Ra' are hydrophobic radicals -Hy,
- n' + m' represents the degree of polymerization DP of the co-polyamino acid, namely the mean number of monomeric units per co-polyamino acid chain and 8 ≤ n' + m' ≤ 50.
- Q is corresponding to only one Q', in particular chosen from the group consisting of Formula A-III, A-IV and A-Iva 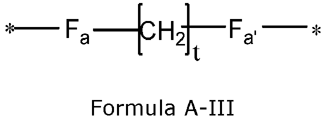
- wherein 1 ≤ t ≤ 8, in particular with Fa and Fa' being -NH-, 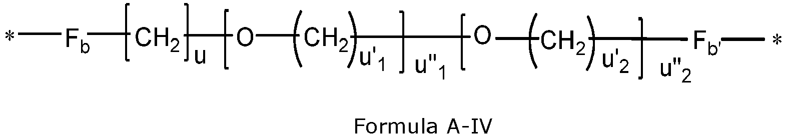
wherein:
- at least one of u₁" or u₂" is different to 0.
- If u₁*"* ≠ 0 then u₁' ≠ 0 and if u₂" ≠ 0 then u₂' ≠ 0,
- u₁' and u₂' are identical or different and,
- 2 ≤ u ≤ 4,
- 0 ≤ u₁' ≤ 4,
- 0 ≤ u₁" ≤ 8, in particular 0 ≤ u₁" ≤ 4,
- 0 ≤ u₂' ≤ 4
- 0 ≤ u₂" ≤ 8, in particular 0 ≤ u₂" ≤ 4,
and
*-Fb-(CH₂)₂-(O(CH₂)₂)aa -Fb' Formula A-IVa
wherein
- aa is an integer comprised between 1 and 16, in particular between 2 and 8, and
- Fb and Fb', identical or different, are representing functions -NH- or -CO-, in particular Fb and Fb' represents -NH-.

8. The composition according to any of claims 1 to 7, **characterized in that** concentration of co-polyamino acid bearing carboxylate charges and hydrophobic radicals is at most 60 mg/ml.

9. The composition according to any of claims 1 to 8, **characterized in that** it further comprises a preservative.

10. The composition according to any of claims 1 to 6, 7 and 8, **characterized in that** said composition is in the form of an aqueous injectable solution, whose pH is comprised from 5.0 to 6.0, comprising
- semaglutide,
- cagrilintide, and
- a co-polyamino acid (PLG) bearing carboxylate charges and hydrophobic radicals Hy having the following Formula: 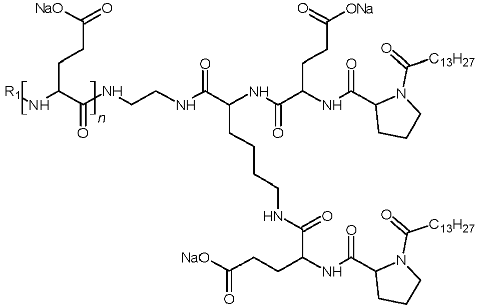
M = 0.02 to 0.1, n = 10 to 50, R₁ = H or pyroglutamate.

11. The composition according to any of claims 1 to 10, for use as a medicament.

12. The composition according to any of claims 1 to 11, for use for treatment of overweight, obesity, diabetes, cardio-vascular disease(s), non-alcoholic steatohepatitis (NASH), and/or cognitive impairment, such as caused by Alzheimer Disease (AD).

13. Injection device comprising a composition such as disclosed in any one of the claims 1 to 12.
